(19) Europäisches Patentamt
European Patent Office
Office européen des brevets

(11) EP 1 173 413 B1

(12) EUROPEAN PATENT SPECIFICATION

(45) Date of publication and mention of the grant of the patent:
**30.06.2004 Bulletin 2004/27**

(21) Application number: **00927038.0**

(22) Date of filing: **25.04.2000**

(51) Int Cl.7: **C07C 271/22**, A61P 43/00, A61K 31/325

(86) International application number:
**PCT/EP2000/003688**

(87) International publication number:
**WO 2000/064863 (02.11.2000 Gazette 2000/44)**

(54) **USE OF 2,4-DIAMINO-3-HYDROXYCARBOXYLIC ACID DERIVATIVES AS PROTEASOME INHIBITORS**

2,4-DIAMINO-3-HYDROXYCARBOXYLSÄUREDERIVATE ALS PROTEASOME INHIBITOREN

DERIVES D'ACIDE 2,4-DIAMINO-3-HYDROXYCARBOXYLIQUE UTILISES COMME INHIBITEURS DE LA PROTEASOME

(84) Designated Contracting States:
**AT BE CH CY DE DK ES FI FR GB GR IE IT LI LU MC NL PT SE**

(30) Priority: **27.04.1999 US 300779**
**02.09.1999 US 388700**

(43) Date of publication of application:
**23.01.2002 Bulletin 2002/04**

(73) Proprietors:
- **Novartis AG**
**4056 Basel (CH)**
Designated Contracting States:
**BE CH DE DK ES FI FR GB GR IE IT LI LU MC NL PT SE CY**
- **Novartis Pharma GmbH**
**1230 Wien (AT)**
Designated Contracting States:
**AT**

(72) Inventors:
- **FRANCE, Dennis**
**Morris Plains, NJ 07950 (US)**
- **FÜRST, Peter**
**CH-4310 Rheinfelden (CH)**
- **ZIMMERMANN, Johann**
**D-79424 Auggen (DE)**
- **GARCIA-ECHEVERRIA, Carlos**
**CH-4052 Basel (CH)**
- **SCHOLZ, Dieter**
**A-1040 Wien (AT)**
- **FURET, Pascal**
**F-68800 Thann (FR)**
- **IMBACH, Patricia**
**CH-4303 Kaiseraugst (CH)**

(74) Representative: **Grubb, Philip William et al**
**Novartis AG,**
**Corporate Intellectual Property,**
**Lichtstrasse 35**
**4002 Basel (CH)**

(56) References cited:
**EP-A- 0 615 969** **WO-A-93/01166**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 173 413 B1

## Description

The present invention relates to a new use of 2,4-diamino-3-hydroxycarboxylic acids of formula I in which the symbols and substituents have the meaning as given hereinafter in free form or in pharmaceutically acceptable salt or complex form, said compound group being referred to hereinafter collectively as COMPOUNDS OF THE INVENTION, in the manufacture of a pharmaceutical composition for the treatment of a proliferative disease, e.g. of a solid tumor; to new 2,4-diamino-3-hydroxycarboxylic acids and their pharmaceutically acceptable salts; and to a pharmaceutical composition comprising a COMPOUND OF THE INVENTION together with a pharmaceutical carrier.

Proteins targeted for the degradation by the multicatalytic proteasome complex are known to have inter alia functions in the cell-cycle control (e.g. cyclins, p21, p27) and apoptosis (e.g. p53) (Rolfe, M., Chiu, I.M. and Pagano, M., The ubiquitin-mediated proteolytic pathway as therapeutic area. J. Mol. Med. 75, 1997, 5-17). Inhibitors of the proteasome are therefore suitable for the treatment of proliferative diseases which respond to the inhibition of the proteasome activity. Proliferative diseases like psoriaris and in particular solid tumors like colon tumor, breast tumor, lung tumor and prostate tumor can be mentioned here.

The present invention relates to a new use of 2,4-diamino-3-hydroxycarboxylic acids of formula I,

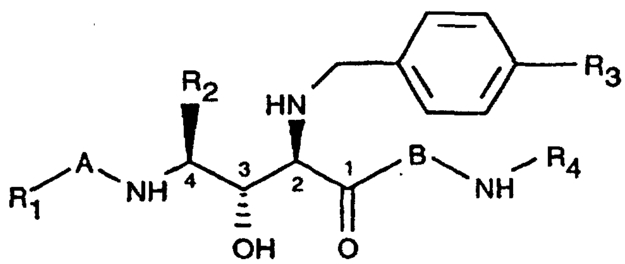

(I)

wherein

- A and B independently represent a bond or an unsubstituted or substituted aminoacyl moiety;
- $R_1$ represents hydrogen; an amino protecting group; or a group of formula $R_5Y$- wherein
  - $R_5$ represents hydrogen or an unsubstituted or substituted alkyl, alkenyl, alkinyl, aryl, arylalkyl, heteroaryl, heteroarylalkyl, heterocyclyl or heterocyclylalkyl group; and
  - Y represents -CO-; -NH-CO-; -NH-CS-; $-SO_2-$; -O-CO-; or -O-CS-;
- $R_2$ represents the side chain of a natural amino acid; an alkyl, arylalkyl, heteroarylalkyl or cycloalkylalkyl group; or trimethylsilylmethyl, 2-thienylmethyl or styrylmethyl;
- $R_3$ represents halogen, alkyl, alkoxy or hydroxyalkoxy; and
- $R_4$ represents 2(R)-hydroxyindan-1(S)-yl; (S)-2-hydroxy-1-phenylethyl; or 2-hydroxybenzyl unsubstituted or substituted in 4 position by methoxy;

wherein the 2,4-diamino-3-hydroxycarboxylic acid is in free form or is a pharmaceutically acceptable salt thereof in the manufacture of a pharmaceutical composition for the treatment of a proliferative disease responsive to an inhibition of the multicatalytic proteasome complex.

Unsubstituted or substituted alkyl preferably is alkyl of 1 to 5 carbon atoms, preferably of 1 to 4 carbon atoms, e.g. methyl, ethyl, isopropyl or tert-butyl; it is especially of 1 or 4 carbon atoms. The substituent is e.g. phenoxy, hydroxy or unprotected or protected amino.

Unsubstituted or substituted arylalkyl is e.g. phenylalkyl of altogether 7 to 10 carbon atoms, such as benzyl or 2-phenylethyl. It is unsubstituted or substituted in the aryl or alkyl moiety by e.g. hydroxy, such as in benzyl-CH(OH)- or phenyl-$CH(CH_2OH)$-, by alkyl, amino or alkylamino; or is e.g. naphthylalkyl of 1 to 4 carbon atoms in the alkylene part, especially naphthylmethyl.

An amino protecting group preferably is benzyloxycarbonyl, cycloalkylalkoxycarbonyl, especially cyclohexylmethoxycarbonyl, or tert-butoxycarbonyl. Unsubstituted or substituted heteroarylalkyl preferably is pyridylalkyl, especially 2-pyridylmethyl and 4-pyridylmethyl.

Aryl, heteroaryl and the aryl parts of arylalkyl and heteroarylalkyl may be mono- or polycyclic, such as e.g. pyridyl, naphthyl or benzimidazolyl. The alkylene part of arylalkyl or heteroarylalkyl may be substituted by e.g. hydroxy.

A heterocyclyl group, and the heterocyclyl part of a heterocyclylalkyl group, is a saturated heterocyclic group

having one or more heteroatoms selected from nitrogen, oxygen and sulfur. It preferably has 5 or 6 ring constitutent atoms, and preferably up to 3 heteroatoms.

Cycloalkylalkyl preferably is cyclohexylalkyl; it preferably is of 1 to 4 carbon atoms in the alkylene part.

Halogen is fluorine, chlorine, bromine or iodine, preferably chlorine or bromine.

Alkyl and alkoxy preferably are of 1 to 4 carbon atoms, especially of 1 or 2 carbon atoms, more especially methyl or methoxy.

Hydroxyalkoxy preferably is ω-hydroxyalkoxy of 2 to 4 carbon atoms, especially 2-hydroxyethoxy.

A salt is e.g. an acid addition salt such as a hydrochloride.

The compounds of formula I have several chiral centers and can therefore exist in a variety of stereoisomers. The invention provides all stereoisomers as well as racemic mixtures unless specified otherwise. The isomers may be resolved or separated by conventional techniques, e.g. chromatographically. As appears from formula I the configuration at the carbon atom in the 2 position is R, in the 3 and 4 positions it is S.

$R_1$ preferably is hydrogen, pyridylalkoxycarbonyl, naphthylalkoxycarbonyl, naphthylalkylcarbonyl, benzyl-CH (OH)-carbonyl, phehoxymethylcarbonyl, phenylalkylcarbonyl or an amino protecting group such as tert.-butoxycarbonyl, cycloalkylalkoxycarbonyl, especially cyclohexylmethoxycarbonyl, or benzyloxycarbonyl which is unsubstituted or substituted by alkyl or amino; it especially is naphthylmethoxycarbonyl, naphthylmethylcarbonyl, pyridylmethoxycarbonyl, phenylpropionyl, aminophenylpropionyl, tert.-butoxycarbonyl, aminobenzyloxycarbonyl, alkylbenzyloxycarbonyl, dialkylbenzyloxycarbonyl or benzyloxycarbonyl, even more preferably benzyloxycarbonyl.

When A is an unsubstituted or substituted aminoacyl moiety, it preferably is an unsubstituted or substituted α-aminoacyl moiety such as alanine, leucine, isoleucine, asparagine, valine, tert-butylglycine, tert-leucine or histidine. It preferably is the protected or unprotected moiety of a natural α-amino acid, preferably of an amino acid which is a normal constitutive part of proteins, or tert-leucine. It preferably has the L configuration. A is especially glycine, L-valine, L-tert-leucine or a bond, even more preferably L-tert-leucine.

$R_2$ preferably is the side chain of a natural amino acid, preferably of an α-amino acid, preferably of an amino acid which is a normal constitutive part of proteins. It is e.g. isopropyl, aminocarbonylmethyl, methyl, 1-methylpropyl, benzyl, 4-hydroxybenzyl or isobutyl, preferably benzyl.

When B is an unsubstituted or substituted aminoacyl moiety, it preferably is an unsubstituted or substituted α-aminoacyl moiety, such as phenylalanine, valine, leucine, isoleucine, alanine or asparagine. It preferably is the unsubstituted or substituted moiety of a natural α-amino acid, preferably of an amino acid which is a normal constitutive part of proteins. α-Amino acids with a second carboxyl group, e.g. glutaminic acid, are preferably esterified with an $C_1$-$C_3$-alcohol, especially methanol. It preferably has the L configuration. B especially is L-valine, L-glutaminic acid methyl ester or a bond, even more preferably L-valine.

$R_3$ preferably is halogen, methyl or methoxy, especially methoxy.

$R_4$ preferably is 2(R)-hydroxyindan-1(S)-yl or 2-hydroxybenzyl unsubstituted or substituted as defined above, especially 2-hydroxy-4-methoxy-benzyl.

Y preferably is -CO- or -O-CO-, especially -O-CO-.

$R_5$ preferably is an unsubstituted or substituted alkyl, arylalkyl or heteroarylalkyl group, especially alkyl; when it is unsubstituted or substituted heteroarylalkyl it preferably is pyridylalkyl, especially 2-pyridylmethyl; when it is unsubstituted or substituted arylalkyl it preferably is benzyl-CH(OH)-; when it is substituted alkyl it preferably is phenoxymethyl.

The COMPOUNDS OF THE INVENTION are described in the patent applications EP 94810150.6 and JP 041047/94 and the patents AU 672867, EC 961021, TW NI-74341 and US 5,538,997 to be active against AIDS due to their capability to inhibit the HIV proteinase.

In accordance with the present invention it has now been found that COMPOUNDS OF THE INVENTION surprisingly have a beneficial effect on proliferative diseases, e.g. on psoriaris and in particular on solid tumors like colon tumor, breast tumor, lung tumor and prostate tumor.

The COMPOUNDS OF THE INVENTION may be prepared by a process as described in the above cited patents and patent applications. Especially, the compounds of Table 1 can be prepared by the procedures described below (reference is made to US 5,538,997).

Abbreviations:

| | |
|---|---|
| Bn | benzyl |
| BOC | tert.-butoxycarbonyl |
| ES-MS | electron spray mass spectrometry |
| Glu | glutaminic acid |

| | |
|---|---|
| Gly | glycine |
| HPLC | high pressure liquid chromatography |
| Me | methyl |
| TFA | trifluoroacetic acid |
| tLeu | tert.-leucine |
| Val | valine |
| Z | benzyloxycarbonyl |

Table 1

| ($R_2$ = Bn, $R_3$ = OMe, $R_4$ = 2-OH, 4-MeO-Bn) | | | |
|---|---|---|---|
| Example | A | B | $R_1$ |
| 1 | L-tLeu | L-Val | Z |
| 2 | L-Val | O-Me-L-Glu | Z |
| 3 | L-tLeu | L-Val | BOC |
| 4 | L-tLeu | L-Val | naphth-1-yl methoxycarbonyl |
| 5 | L-tLeu | L-Val | naphth-2-yl methoxycarbonyl |
| 6 | L-tLeu | L-Val | 4-pyridyl methoxycarbonyl |
| 7 | Gly | L-Val | Z |
| 8 | L-Val | L-Val | Z |
| 9 | L-tLeu | L-tLeu | Z |
| 10 | L-tLeu | L-Val | naphth-1-yl-acetyl |
| 11 | L-tLeu | L-Val | 3-methylbenzyloxycarbonyl |
| 12 | L-tLeu | L-Val | 3-(3-aminophenyl)propionyl |
| 13 | L-tLeu | L-Val | 3-aminobenzyloxycarbonyl |
| 14 | L-tLeu | L-Val | 3,5-dimethylbenzyloxy-carbonyl |
| 15 | L-tLeu | L-Val | cyclohexylmethoxycarbonyl |

Examples 1-15 are prepared as described in the general reaction scheme of US 5,538,997 for the conversion of compounds of formula III to compounds of formula VII. The synthesis of the different building blocks is described in the above mentioned US patent under Examples 1 to 4, col. 6 to 8, and 'Further Intermediates', col. 9 to 11.

The purity of the compounds is verified by reversed-phase analytical HPLC on a Nucleosil C18-column (250 x 4 mm, 5 mm, 100 Å): System A: linear gradient over 7 min of MeCN/0.09% TFA and $H_2O$/0.1% TFA from 1:49 to 1:0 and 3 min at 1:0; flow rate 2.0 mL/min, detection at 215 nm; System B: linear gradient over 10 min of MeCN/0.09% TFA and $H_2O$/0.1% TFA from 1:49 to 1:0; flow rate 2.0 mUmin, detection at 215 nm. System C: reversed-phase analytical HPLC on a Nucleosil $C_{18AB}$-column (125 x 3 mm, 3 µm, 120 Å); linear gradient over 7 min of MeCN/0.09% TFA and $H_2O$/0.1% TFA from 1:49 to 1:0 and 3 min at 1:0; flow rate 1.15 mL/min. detection at 215 nm.

Example 1: N-{4-(S)-[N-Benzyloxycarbonyl-tert-leucinoyl)amino]-3-(S)-hydroxy-2(R)-(4-methoxybenzylamino)-5-phenyl]pentanoyl-L-valine-N-[(2-hydroxy-4-methoxy)benzyl]amide

The compound of Example 1 corresponds to the compound of Example 4 of US 5,538,997.

Example 2: 4-[4-(2-Benzyloxycarbonylamino-3-methyl-butyrylamino)-3-hydroxy-2-(4-methoxy-benzylamino)-5-phenyl-pentanoylamino]-4-(2-hydroxy-4-methoxy-benzylcarbamoyl)-butyric acid methyl ester

The title compound is prepared analogously to Example 4 of US 5,538,997. The building blocks are prepared by using BOC-L-glutamic acid γ-methylester α-p-nitrophenyl-ester instead of BOC-L-valine p-nitrophenylester in step C.c) and BOC-L-valine instead of BOC-L-tert-leucine in step B) under 'Further Intermediates'; ES-MS 856 [M + $H^+$].

Example 3: {1-[1-Benzyl-2-hydroxy-3-[1-(2-hydroxy-4-methoxy-benzylcarbamoyl)-2-methyl-propylcarbamoyl]-3-(4-methoxy-benzylamino)-propylcarbamoyl]-2,2-dimethyl-propyl}-carbamic acid tert-butyl ester

The compound of Example 3 corresponds to the compound of Example 11 of US 5,538,997.

Example 4: {1-[1-Benzyl-2-hydroxy-3-[1-(2-hydroxy-4-methoxy-benzyl-carbamoyl)-2-methyl-propylcarbamoyl]-3-(4-methoxy-benzylamino)-propylcarbamoyl]-2,2-dimethyl-propyl}-carbamic acid naphthalen-1-ylmethyl ester

The title compound is prepared analogously to Example 4 of US 5,538,997 by using carbonic acid naphthalen-1-ylmethyl ester 4-nitro-phenyl ester instead of N-(benzyloxy-carbonyloxy)-succinimide. Carbonic acid naphthalen-1-ylmethyl ester 4-nitro-phenyl ester is synthesised starting from 1-napthalenemethanol (Aldrich, Buchs, Switzerland) according to a procedure known in the art (P. Furet et al., J. Med. Chem. 1997, 40, 3551-3556). ES-MS (title compound): 877.3 [M + $H^+$], 899.4 [M + $Na^+$]; HPLC (System A) produced a single peak at $t_R$= 7.30 min.

Example 5: {1-[1-Benzyl-2-hydroxy-3-[1-(2-hydroxy-4-methoxy-benzylcarbamoyl)-2-methyl-propylcarbamoyl]-3-(4-methoxy-benzylamino)-propylcarbamoyl]-2,2-dimethyl-propyl]-carbamic acid naphthalen-2-ylmethyl ester

The title compound is prepared analogously to Example 4 of US 5,538,997 by using carbonic acid naphthalen-2-ylmethyl ester 4-nitro-phenyl ester instead of N-(benzyloxy-carbonyloxy)-succinimide. Carbonic acid naphthalen-2-ylmethyl ester 4-nitro-phenyl ester is synthesised starting from 2-napthalenemethanol (Aldrich, Buchs, Switzerland) according to a procedure known in the art (P. Furet et al., J. Med. Chem. 1997, 40, 3551-3556). ES-MS (title compound): 877.3 [M + $H^+$], 899.4 [M + $Na^+$]; HPLC (System A) produced a single peak at $t_R$= 7.38 min.

Example 6: {1-[1-Benzyl-2-hydroxy-3-[1-(2-hydroxy-4-methoxy-benzylcarbamoyl)-2-methyl-propylcarbamoyl]-3-(4-methoxy-benzylamino)-propylcarbamoyl]-2,2-dimethyl-propyl}-carbamic acid pyridin-4-ylmethyl ester

The title compound is prepared analogously to Example 4 of US 5,538,997 by using carbonic acid 4-nitro-phenyl ester pyridin-4-ylmethyl ester instead of N-(benzyloxycarbonyloxy)-succinimide. Carbonic acid 4-nitro-phenyl ester pyridin-4-ylmethyl ester is synthesised starting from 4-(hydroxymethyl)pyridine (Fluka, Buchs, Switzerland) according to a procedure known in the art (P. Furet et al., J. Med. Chem. 1997, 40, 3551-3556). ES-MS (title compound): 828.3 [M + $H^+$], 849.2 [M + $Na^+$]; HPLC (System B) produced a single peak at $t_R$= 6.80 min.

Example 7: {[1-Benzyl-2-hydroxy-3-{1-(2-hydroxy-4-methoxy-benzylcarbamoyl)-2-methyl-propylcarbamoyl]-3-(4-methoxy-benzylamino)-propylcarbamoyl]-methyl}-carbamic acid benzyl ester

The title compound is prepared analogously to Example 4 of US 5,538,997. One building block is prepared by using Z-L-glycine instead of BOC-L-tert-leucine in step B) under'Further Intermediates'. ES-MS: 771.0 [M + $H^+$]; HPLC (System B) produced a single peak at $t_R$= 9.45 min.

Example 8: {1-[1-Benzyl-2-hydroxy-3-[1-(2-hydroxy-4-methoxy-benzylcarbamoyl)-2-methyl-propylcarbamoyl]-3-(4-methoxy-benzylamino)-propylcarbamoyl]-2-methyl-propyl}-carbamic acid benzyl ester

The compound of Example 8 corresponds to the compound of Example 22 of US 5,538,997. ES-MS: 813.0 [M + $H^+$]; HPLC (System A), single peak at $t_R$= 7.53 min.

Example 9: {1-[1-Benzyl-2-hydroxy-3-[1-(2-hydroxy-4-methoxy-benzylcarbamoyl)-2,2-dimethyl-propylcarbamoyl]-3-(4-methoxy-benzylamino)-propylcarbamoyl]-2,2-dimethyl-propyl)-carbamic acid benzyl ester

The title compound is prepared analogously to Example 4 of US 5,538,997. One building block is prepared by using BOC-L-tert-leucine p-nitrophenylester instead of BOC-L-valine p-nitrophenylester in step C.c) under'Further Intermediates'. ES-MS (title compound): 841.0 [M + $H^+$]; HPLC (System A) produced a single peak at $t_R$= 7.22 min.

Example 10: 4-[3,3-Dimethyl-2-(2-naphthalen-1-yl-acetylamino)-butyrylamino]-3-hydroxy-2-(4-methoxy-benzylamino)-5-phenyl-pentanoic acid [1-(2-hydroxy-4-methoxy-benzyl-carbamoyl)-2-methyl-propyl]-amide

The title compound is prepared analogously to Example 4 of US 5,538,997, but using 1-naphthylacetic acid (Fluka, Buchs, Switzerland) to incorporate the final N-terminal group $R_1$. 1-Naphthylacetic acid (1.1 equiv.) is coupled first with 2-(2-pyridon-1-yl)-1,1,3,3-tetra-methyluronium-tetrafluoroborate (1.1 equiv.; Fluka) in the presence of diisopropylethylamine (2.2 equiv.) and using N,N-dimethylacetamide as solvent. ES-MS (title compound): 861.0 [M + $H^+$], 883.1 [M + $Na^+$]; HPLC (System A) produced a single peak at $t_R$=7.17 min.

Example 11: {1-[1-Benzyl-2-hydroxy-3-[1-(2-hydroxy-4-methoxy-benzylcarbamoyl)-2-methyl-propylcarbamoyl]-3-(4-methoxy-benzylamino)-propylcarbamoyl]-2,2-dimethyl-propyl}-carbamic acid 3-methylbenzyl ester

The title compound is prepared analogously to Example 4 of US 5,538,997 by using carbonic acid 3-methyl-benzyl ester 4-nitro-phenyl ester instead of N-(benzyloxycarbonyloxy)-succinimide. Carbonic acid 3-methyl-benzyl ester 4-nitro-phenyl ester is synthesised starting from 3-methylbenzyl alcohol (Aldrich, Buchs, Switzerland) according to a procedure known in the art (P. Furet et al., J. Med. Chem. 1997,40, 3551-3556). ES-MS (title compound): 841.0 [M + $H^+$], 843.0 [M + $Na^+$]; HPLC (System A): single peak at $t_R$= 7.17 min.

Example 12: 4-{2-[3-(3-Amino-phenyl)-propionylamino]-3,3-dimethyl-butyrylamino}-3-hydroxy-2-(4-methoxy-benzylamino)-5-phenyl-pentanoic acid [1-(2-hydroxy-4-methoxy-benzylcarbamoyl)-2-methyl-propyl]-amide

The title compound is prepared analogously to Example 4 of US 5,538,997, but using 3-(3-tert.-butoxycarbonylamino-phenyl)-propionic acid to incorporate the final N-terminal group $R_1$. 3-(3-tert.-Butoxycarbonylamino-phenyl)-propionic acid is synthesised starting from 3-(3-aminophenyl)propionic acid (Trans World Chemicals, Inc., Rockville, U.S.A.) according to a procedure known in the art (P. Furet et al., J. Med. Chem. 1997, 40, 3551-3556) and incorporated at the N-terminus using a protocol analogous to Example 10. The tert.-butoxy-carbonyl group is removed by treatment with trifluoroacetic acid. ES-MS (title compound): 840.0 [M + $H^+$], 862.1 [M + $Na^+$]; HPLC (System A): single peak at $t_R$= 5.74 min.

Example 13: {1-[1-Benzyl-2-hydroxy-3-[1-(2-hydroxy-4-methoxy-benzylcarbamoyl)-2-methyl-propylcarbamoyl]-3-(4-methoxy-benzylamino)-propylcarbamoyl]-2,2-dimethyl-propyl}-carbamic acid 3-amino-benzyl ester

The title compound is prepared analogously to Example 4 of US 5,538,997 by using carbonic acid 3-tert.-butoxycarbonylamino-benzyl ester 4-nitro-phenyl ester instead of N-(benzyloxycarbonyloxy)-succinimide. Carbonic acid 3-tert.-butoxycarbonylamino-benzyl ester 4-nitro-phenyl ester is synthesised starting from 3-aminobenzyl alcohol (Aldrich) according to a procedure known in the art (P. Furet et al., J. Med. Chem. 1997, 40, 3551-3556). The tert.-butoxycarbonyl group is removed by treatment with trifluoroacetic acid. ES-MS (title compound): 842.0 [M + $H^+$], 864.0 [M + $Na^+$], $t_R$= 5.85 min (HPLC, System A).

Example 14: {1-[1-Benzyl-2-hydroxy-3-[1-(2-hydroxy-4-methoxy-benzyl-carbamoyl)-2-methyl-propylcarbamoyl]-3-(4-methoxy-benzylamino)-propylcarbamoyl]-2,2-dimethyl-propyl}-carbamic acid 3.5-dimethyl-benzyl ester

The title compound is prepared analogously to Example 4 of US 5,538,997 by using carbonic acid 3,5-dimethyl-benzyl ester 4-nitro-phenyl ester instead of N-(benzyloxy-carbonyloxy)-succinimide. Carbonic acid 3,5-dimethyl-benzyl ester 4-nitro-phenyl ester is synthesised starting from 3,5-dimethylbenzyl alcohol (Aldrich, Switzerland) according to a procedure known in the art (P. Furet et al., J. Med. Chem. 1997, 40, 3551-3556). ES-MS (title compound): 855.0 [M + $H^+$], 877.0 [M + $Na^+$], $t_R$= 7.32 min (HPLC, System A).

Example 15: {(S)-1-[(1S,2S,3R)-1-Benzyl-2-hydroxy-3-[(S)-1-(2-hydroxy-4-methoxy-benzylcarbamoyl)-2-methyl-propylcarbamoyl]-3-(4-methoxy-benzylamino)-propylcarbamoyl]-2,2-dimethyl-propyl}-carbamic acid cyclohexylmethyl ester

The title compound is prepared analogously to Example 4 of US 5,538,997 by using carbonic acid 3,5-dimethyl-benzyl ester 4-nitro-phenyl ester instead of N-(benzyloxy-carbonyloxy)-succinimide. Carbonic acid cyclohexylmethyl ester 4-nitro-phenyl ester (1.1 equiv.) is synthesised starting from cyclohexyl-methanol (Fluka, Buchs, Switzerland) according to a procedure known in the art (P. Furet et al., J. Med. Chem. 1997, 40, 3551-3556). ES-MS (title compound): 833.0, $t_R$= 7.55 min (System C).

Example 16: Composition for oral Application

20.0 g of a solution for oral application can be prepared as follows (% means weight ingredient / total weight solution):

| | |
|---|---|
| Cremophor RH 40<br>cornoil-mono-di-tri-glycerides<br>propylene glycol<br>compound of formula I | 9.6 g (48 %),<br>5.8 g (29 %),<br>3.8 g (19 %),<br>0.8 g ( 4 %). |

The solution is kept at room temperature until use.

The utility of the COMPOUNDS OF THE INVENTION in treating the proliferative diseases mentioned above for example may be demonstrated in the test methods described hereinafter.

Inhibition of 20S Proteasome

The COMPOUNDS OF THE INVENTION inhibit the 20S proteasome.

The multicatalytic proteasome complex is responsible for the ATP-dependent proteolysis of most cellular proteins. Although the 20S proteasome contains the proteolytic core, it cannot degrade proteins *in vivo* unless it is complexed with 19S caps, at either end of its structure, which itself contains multiple ATPase activities. This larger structure is known as the 26S proteasome and will rapidly degrade proteins that have been targeted for the degradation by the addition of multiple molecules of the 8.5 kDa polypeptide ubiquitin. Proteins targeted for proteasomal degradation have functions in the cell-cycle control (e.g. cyclins, p21, p27) and apoptosis (e.g. p53) (Rolfe, M., Chiu, I.M. and Pagano, M., see above). The COMPOUNDS OF THE INVENTION are therefore highly suitable for the treatment of diseases which respond to inhibition of the activity of the 20S proteasome, which is the case for the proliferative diseases mentioned above.

The inhibition of the chymotrypsin-like activity of the 20S proteasome can be demonstrated by the following experiment. It is based on the hydrolysis of the fluorogenic peptide Suc-LLVY-AMC (Succinyl-leucine-leucine-valine-tyrosine-7-amino-4-methyl-coumarin), which is cleaved exclusively at the Y-AMC bond by the 20S proteasome. Hydrolysis of this peptide is accompanied by an increase in fluorescence intensity ($\lambda_{ex}$ 355 nm, $\lambda_{em}$ 460 nm) due to release of the internally quenched 2-aminobenzoyl fluorescence that accompanies diffusion apart of the hydrolysis product Suc-LLVY.

2 µl of a 1 mM solution of a test compound in DMSO (dimethylsulfoxide, 20 µM final concentration in the well) are preincubated for 60 min at room temperature in black 96-well microtiter plates together with a mixture of 1 µl purified human placenta 20S proteasome (Diabetes Forschungsinstitut, Düsseldorf, Germany; about 100 ng proteasome, depending on the proteasome preparation) and 47 µl buffer-Ca (5 mM $CaCl_2$, 20 mM Tris/HCl (Tris-(hydroxymethyl)-amino-methane hydrochloride) pH 8.0). 3 µl of a 2.67 mM solution of Suc-LLVY-AMC (Bachem, Switzerland) in DMSO (80 µM final concentration in the well) and 47 µl buffer-Ca are mixed and added. The resulting mixture is incubated for 3 - 24 h at 37 °C. If desired, proteolysis of the substrate can be stopped by addition of 50 µl stop solution (100 mM monochloroacetic acid, 130 mM NaOH, 100 mM acetic acid, pH = 4.3). The fluorescence is monitored with a FLUOROSKAN ASCENT™ microtiter plate reader.

To each measure series two control experiments have to be carried out:

1.) 0% value: 2 µl DMSO are used in the above described assay instead of 2 µl of a 1 mM solution of a test compound in DMSO and 48 µl buffer-Ca instead of a mixture of 47 µl buffer-Ca and 1 µl purified proteasome.
2.) 100% value: in the above described assay 2 µl DMSO are used instead of 2 µl of a 1 mM solution of the test compound in DMSO.

Calculation

$$\text{\% remaining activity} = \frac{(\text{value with compound - 0\% value})}{(\text{100\% value - 0\% value})} \cdot 100\%$$

The $IC_{50}$ value is defined as that concentration of a compound at which the remaining activity is 50 %. Exemplary $IC_{50}$ values determined in this test for the COMPOUNDS OF THE INVENTION are given below (Table 2).

Table 2

| ($R_2$ = Bn, $R_3$ = OMe, $R_4$ = 2-OH, 4-MeO-Bn) | | | | |
|---|---|---|---|---|
| Example | A | B | $R_1$ | $IC_{50}$ [μM] |
| 1 | L-tLeu | L-Val | Z | 0.9 |
| 2 | L-Val | O-Me-L-Glu | Z | 0.4 |
| 3 | L-tLeu | L-Val | BOC | 26 |
| 4 | L-tLeu | L-Val | naphth-1-yl methoxycarbonyl | 2 |
| 5 | L-tLeu | L-Val | naphth-2-yl methoxycarbonyl | 5.2 |
| 6 | L-tLeu | L-Val | 4-pyridyl methoxycarbonyl | 2.2 |
| 7 | Gly | L-Val | Z | 9 |
| 8 | L-Val | L-Val | Z | 3.3 |
| 10 | L-tLeu | L-Val | naphth-1-yl-acetyl | 0.1 |
| 11 | L-tLeu | L-Val | 3-methylbenzyl-oxycarbonyl | 0.5 |
| 12 | L-tLeu | L-Val | 3-(3-amino-phenyl)propionyl | 0.6 |
| 13 | L-tLeu | L-Val | 3-aminobenzyl-oxycarbonyl | 0.6 |
| 14 | L-tLeu | L-Val | 3,5-dimethyl-benzyloxy-carbonyl | 1.1 |
| 15 | L-tLeu | L-Val | cyclohexylmethoxycarbonyl | 1.0 |

The antitumoral action of the COMPOUNDS OF THE INVENTION can also be demonstrated *in vivo*:

*In vivo* evaluation of antitumor action in nude mice using human tumor xenografts

Female or male BALB/c *nu*/*nu* (Novartis animal farm, Sisseln, Switzerland or Bom-holtgaard, Copenhagen, Denmark) mice with subcutaneously transplanted human tumors are used for the evaluation of the antitumor action of the COMPOUNDS OF THE INVENTION against cell lines originating from the four tumor types, breast tumor: MCF-7; lung tumor: NCI H596: colon tumor: HCT 116; and prostate tumor: PC 3.

Materials:

Human colon carcinoma HCT 116 (ATCC CCL 247), human squamous cell lung carcinoma NCI H596 (ATCC HTB 178), estrogen-dependent breast carcinoma MCF-7 (ATCC HTB 22), and the human prostate cancer PC 3 cell line are obtained from the American Type Culture Collection (ATCC, Rockville, USA). The cells are cultured at 37°C in a 5 % v/v $CO_2$ and 80 % relative humidity atmosphere in the following media: NCl H 596: RPMI 1640, 20 % v/v FBS (fetal bovine serum), 1 % w/v glutamine; HCT-116: McCoy's 5A, 10 % v/v FBS, 1 % w/v glutamine; PC 3: RPMI 1640, 20 % v/v FBS, 1 % w/v glutamine; MCF-7, RPMI 1640, 20 % v/v FBS, 1 % w/v glutamine. All of these cell lines are adherent and can be released by rinsing with Hank's balanced salt and treatment with 0. 25 % w/v trypsin. All of these cells are prepared as master cell stocks (in culture media supplemented to contain 20 % v/v FBS and 7 % v/v DMSO) and stored at -125°C (liquid nitrogen vapors). Working cell stocks are prepared from the master stocks by thawing and expanding the cells over three passages, which are then distributed to vials and frozen. Viability (trypan blue exclusion test using 0.5 % w/v trypan blue) prior to freezing is > 90 % for all cell lines.

Establishing solid tumors in nude mice:

Mice are kept under controlled conditions (Optimal Hygienic Conditions, [OHC]) with free access to sterile food and water. Tumors are established after subcutaneous injection of cells (a minimum of 2 x $10^6$ cells in 100 μl PBS (phosphate buffered saline) or medium) in carrier mice (4-8 mice per cell line). injections are made s.c. in the left flank of the mouse mid-way between the tail and head. The resulting tumors are serially passaged for a minimum of three consecutive transplantations prior to start of treatment. Tumors are transplanted when the tumor reaches a volume of 800 to 1000 $mm^3$.

Transplanting solid tumors in nude mice:

Donor mice are anesthetized (Forene®, Abbott, Switzerland) and killed by cervical dislocation. The skin is disinfected and the tumor removed by dissection. The outer edges of the tumor mass is removed using a scalpel, and the resulting mass is trimmed into pieces of about 3-4 mm in height. Sections of 3-4 $mm^2$ are cut and placed into sterile 0.9 % w/v NaCl. Sections of tumor that are necrotic are not used.

Tumor fragments are implanted s.c. into the left flank of the recipient mice. Recipient mice are anesthetized (Forene®, Abbott, Switzerland) and the skin on the entire back and sides of the mice is disinfected. The skin 0.5 to 1 cm above the tail is raised and a single 1 to 1.5 cm incision is made. Tumor sections are pushed into a 13-gauge trocar needle. The trocar needle is pushed into the opening of the skin, and advanced under the skin to a point mid-way between the head and the tail. The tumor fragment is deposited by advancing the trocar. The wound is sutured using one or two metal clips.

In the case of estrogen-dependent breast tumors, estrogen pellets (17b-estradiol, 5 mg/ pellet giving a sustained release over 60 days are obtained from Innovative Research of America, Sarasota, USA), are placed subcutaneously in the other flank.

The tumors are allowed to increase until the size is 100 to 150 $mm^3$ before treatment is begun. The tumors are then measured and placed into groups (normally n = 6 to 8) that are balanced according to the mean size and range of tumor volumes. Groups are randomly assigned to treatment groups.

Preparation and application of test compounds

Stock solutions of 40 mg/ml of compound are dissolved in 100 % DMSO and stirred at room temperature until a clear solutions is obtained. Prior to each administration, 10 % Tween 80 (FLUKA, Buchs, Switzerland) is added to the stock solution and then diluted 1:20 (v/v) with sterile 0.9 % w/v NaCl or water. Solutions and dilutions are prepared daily prior to application. Applications are given 7 days a week (p.o., i.p., s.c. or i.v.). The volumes of application are: p.o., 25 ml/kg, i.p., 25 ml/kg, s.c. 10 ml/kg, i.v., 10 ml/kg.

Measurement of tumor volumes

Tumor growth is monitored once, twice or three times weekly (depending on the growth rate of the tumor line) and 24 hours after the last treatment by measuring perpendicular diameters. Calipers capable of determining mm distances are used. Tumor volumes are calculated according to the formula $L \times D^2 \times \pi/6$. Antitumor activity is expressed as T/C % (mean increase of tumor volumes of treated animals divided by the mean increase of tumor volumes of control animals multiplied by 100). Tumor regression (%) represents the smallest mean tumor volume compared to the mean tumor volume at the start of treatment. Delta (Δ) tumor volumes compared the change in tumor volume during the duration of the experiment (volume on the last treatment day - volume on the first treatment day). Any animals in which the tumor reaches a size exceeding approximately 1500 to 2000 $mm^3$ are sacrificed.

Additional measurements

Body weights and survival data are also collected. Delta (Δ) body weights are calculated as an indication of tolerability to treatment (weight on the last treatment day - weight on the first treatment day). Statistically significant body weight loss, or mortalities, are considered indicators of poor tolerability to treatment. Additionally, mice are monitored once or twice daily for general health.

Statistical analyses

The basic approach for statistical analyses is to use tests for multiple comparisons to judge the statistical significance of differences between treatment groups, and differences within a group to determine if treatment induced stable disease or tumor regressions. As subcutaneous tumor volumes are not always normally distributed, differences in the subcutaneous tumor volumes between treatment groups is determined using the non-parametric Kruskal-Wallis one way ANOVA test on ranked data and the statistical significance of differences between treatment groups as compared to control groups determined using the Dunnett test. Pair wise comparisons between all groups is performed using the Student-Newman-Keuls (SNK) method. If only two groups are compared, the Rank sum test is used. Animal body weights are normally distributed, and changes in body weights within a group are analyzed by paired t-tests and between group differences are analyzed by a One-Way ANOVA and pair-wise comparisons made using the Tukey test. For all tests the level of significance is set at $p < 0.05$.

Another *in vivo* test to determine the antitumoral action of the COMPOUNDS OF THE INVENTION is the

following hollow fiber assay:

Hollow Fiber Assay: evaluation of antitumor action in nude mice

In this assay four different human tumors encapsulated in hollow fibers are implantated subcutaneously and/or intraperitonealy into nude mice (athymic female outbred nude mice (Ncr nu/nu)). Animals are then treated with a test compound formulated in an appropriate vehicle, while control animals are treated with the vehicle alone. At the end of the experiment fibers are retrieved, and the number of viable cells is measured using a metabolic assay (MTT, 3-[4,5-dimethylthiazol-2-yl]-2,5-diphenyltetrazolium bromide). Activity of the test compound is measured by comparing the growth of cells in the experimental animals (T) to the growth of cells in the control animals treated with the vehicle alone (C), and is expressed as %T/C.

Materials:

Human colon carcinomas SW 620 and LS 174T, and breast carcinoma MDA-MB-435S are obtained from the American Type Culture Collection (ATCC, Rockville, USA). The colon carcinoma MIP 101, expressing high levels of pgp-1, was originally established from a patient at the Dana Farber Cancer Institute, Boston, USA. All cell lines are grown according to standard tissue culture techniques in RPMI 1640 containing the following additives: 5% FBS (Fetal Bovine Serum), 5 mg/ml insulin, 5 mg/ml transferrin, 5 ng/ml selenous acid, 1 nM β-estradiol, 1 nM testosterone.

Design of the assay:

Four human solid tumor cell lines are encapsulated in PVDF (polyvinylidene fluoride) hollow fibers with 1 mm inner diameter (Biopore, Spectrum Medical, CA, USA). After encapsulation and prior to implantation into animals, cells are allowed to attach to the inner surface of the fiber by incubation in the tissue culture media for 24 hours. Four hollow fibers are then implanted into one animal intraperitoneally or subcutaneously. In the experimental setup four to six animals are used per group, and the experiment consists of a minimum of three groups:

1. "Time 0" group
2. Control (placebo) group
3. "Treated" (tested compound) group.

Treatment starts 24 hours after implantation of the fibers. Animals are treated once daily, at days 1, 3, and 5. At the same time when the treatment starts, the animals from "Time 0" group are sacrificed, fibers are retrieved, and the number of viable cells at the beginning of the experiment is determined ($T_0$). At day 7 after the implantation all animals are sacrificed, fibers are retrieved, and the number of viable cells is determined for the Control ($C_v$), and for the "Treated" ($T_v$) groups. %T/C is then calculated according to the formula:

$$\%T/C = (T_v - T_0)/( C_v - T_0) \times 100\%$$

Encapsulation of tumor cells in hollow fibers:

Fibers are cut into desired length and soaked for at least 72 h in 70% Ethanol. Afterwards, fibers and instruments for the encapsulation are sterilized. The human solid tumor cell line grown in tissue culture is trypsinized, suspended in a small amount of tissue culture media and transferred into the fibers using a syringe. The fibers are heat sealed and incubated at 37 °C for 24 h in an atmosphere comprising 5 % $CO_2$. The fibers are then implantated subcutaneously and/or intraperitonealy into nude mice.

Determination of viable cells in hollow fibers:

1 g MTT is added to 200 ml PBS (phosphate-buffered saline), stirred for 20 min and filtered (0.22 micron filter). 10 ml of this solution are mixed with 40 ml RPMI 1640 with additives to give the MTT working solution. The fibers are incubated in 5 ml RPMI 1640 for 30min at 37 °C in 5% $CO_2$ stabilization. 0.5ml of MTT working solution are added to each well of the sample plate. The plates are incubated at 37 °C in 5% $CO_2$ for 4 h. The MTT is aspirated out of each well in the sample plate. 2 ml of 2.5% aqueous protamine sulfate solution is added to each well of the sample plate. The plates are incubated at 4 °C for 24h. The protamine sulfate is aspirated out of each well in the sample plate. 2 ml of protamine sulfate are added to each well and the plates are incubated at 4 °C for further 2-4 h. Each fiber is

transfered to a well in a 24 well plate. The fibers are cut so that they lie on the bottom of the wells and dried ovemight. 250 µl of DMSO are added to each well. The plates are placed on an orbital shaker for 4 h with a cover to protect the MTT from light. 150 µl of each sample are transfered to the appropriate well in a 96 well plate. The plates are read at 540 nm using DMSO as the blanking well.

As is known from the literature, peptide based drugs characteristically show poor oral bioavailability. This is one of the main obstacles in the development of effective drugs in this peptidic structural class. Surprisingly, the COMPOUNDS OF THE INVENTION show excellent bioavailabilty after oral administration. This can be shown e.g. in the following test:

For peroral administration, a solution of the test substance (25 mg/ml) in a suitable solvent such as Cremophor RH40$^R$ / Maisine$^R$ / propylene glycol / ethanol (38/32/15/15) is prepared. Female Balb/c mice are fasted for 24 hours prior to the start, and throughout the experiment water is given ad libitum. At various times following drug administration, blood samples are obtained by sacrifycing animals under anaesthesia by cutting the vena jugularis, followed by cervical dislocation. Samples are collected in heparinized tubes (typically 0.4-0.6 ml). For sample analysis solid phase extraction and HPLC are used. Drug concentration in the samples is calculated by least-squares linear regression analysis of the peak area ratio (inhibitor/intemal standard) of spiked blood standards versus concentration. From the concentration versus time data, the "Area Under the Curve" (AUC) value is calculated by the trapezoidal rule.

In this test the compounds disclosed in US 5,538,997 exhibit AUC-values of from about 25 µM.h to about 160 µM.h at a dose of 125 mg/kg.

The test shows that the COMPOUNDS OF THE INVENTION can be used as pharmaceuticals.

The invention relates in particular to the use of COMPOUNDS OF THE INVENTION, in which
A and B independently represent a bond or an amino acyl moiety, which is unsubstituted or substituted by alkyl or alkoxycarbonylalkyl;
$R_1$ represents hydrogen; an amino protecting group; or a group of formula $R_5Y$- wherein $R_5$ represents hydrogen or an unsubstituted or substituted alkyl, alkenyl, alkinyl, aryl, arylalkyl, heteroaryl, heteroarylalkyl, heterocyclyl or heterocyclylalkyl group; and
Y represents -CO-; -NH-CO-; -NH-CS-; $-SO_2-$; -O-CO-; or -O-CS-;
$R_2$ represents the side chain of a natural amino acid; an alkyl, arylalkyl, heteroarylalkyl or cycloalkylalkyl group; or trimethylsilylmethyl, 2-thienylmethyl or styrylmethyl;
$R_3$ represents halogen, alkyl, alkoxy or hydroxyalkoxy; and
$R_4$ represents 2(R)-hydroxyindan-1(S)-yl; (S)-2-hydroxy-1-phenylethyl; or 2-hydroxy-benzyl unsubstituted or substituted in 4 position by methoxy.

The invention relates in particular preferably to the use of COMPOUNDS OF THE INVENTION, in which
A and B independently represent a bond or an amino acyl moiety, which is unsubstituted or substituted by alkyl or alkoxycarbonylalkyl;
$R_1$ represents hydrogen; an amino protecting group; or a group of formula $R_5Y$- wherein $R_5$ represents hydrogen or an unsubstituted or substituted alkyl, alkenyl, alkinyl, aryl, arylalkyl, heteroaryl, heteroarylalkyl, heterocyclyl or heterocyclylalkyl group; and
Y represents -CO-; -NH-CO-; -NH-CS-; $-SO_2-$; -O-CO-; or -O-CS-;
$R_2$ represents arylalkyl;
$R_3$ represents halogen, alkyl, alkoxy or hydroxyalkoxy; and
$R_4$ represents 2-hydroxybenzyl unsubstituted or substituted in 4 position by methoxy.

Furthermore, the invention relates preferably to the use of a COMPOUND OF THE INVENTION, in which
A and B independently represent an amino acyl moiety, which is unsubstituted or substituted by $C_1$-$C_4$alkyl or $C_1$-$C_4$alkoxycarbonyl $C_1$-$C_4$alkyl;
$R_1$ represents hydrogen or a group of formula $R_5Y$- wherein
$R_5$ represents hydrogen; $C_1$-$C_4$alkyl which is unsubstituted or substituted by phenoxy, hydroxy or amino; $C_2$-$C_4$alkenyl; $C_2$-$C_4$alkinyl; $C_6$-$C_{10}$aryl; $C_7$-$C_{12}$arylalkyl which is unsubstituted or substituted by hydroxy, $C_1$-$C_4$alkyl, amino or $C_1$-$C_4$alkyl amino; pyridyl $C_1$-$C_4$alkyl; and
Y represents -O-CO- or -CO-;
$R_2$ represents $C_7$-$C_{12}$arylalkyl;
$R_3$ represents halogen, $C_1$-$C_4$alkyl, $C_1$-$C_4$alkoxy or hydroxy $C_1$-$C_4$alkoxy; and
$R_4$ represents 2-hydroxybenzyl which is unsubstituted or substituted in 4 position by methoxy.

More preferably, the invention relates to the use of a COMPOUND OF THE INVENTION, in which
A and B independently represent an amino acyl moiety, which is unsubstituted or substituted by $C_1$-$C_4$alkyl or $C_1$-$C_4$alkoxycarbonyl $C_1$-$C_4$alkyl;
$R_1$ represents hydrogen or a group of formula $R_5Y$- wherein
$R_5$ represents hydrogen; $C_1$-$C_4$alkyl which is unsubstituted or substituted by phenoxy, hydroxy or amino; $C_2$-$C_4$alkenyl; $C_2$-$C_4$alkinyl; $C_6$-$C_{10}$aryl; $C_7$-$C_{12}$arylalkyl which is unsubstituted or substituted by hydroxy, $C_1$-$C_4$alkyl, amino or

$C_1$-$C_4$alkyl amino; pyridyl $C_1$-$C_4$alkyl; and
Y represents -O-CO- or -CO-;
$R_2$ represents $C_7$-$C_{12}$arylalkyl;
$R_3$ represents $C_1$-$C_4$alkoxy; and
$R_4$ represents 2-hydroxybenzyl substituted in 4 position by methoxy.

In a preferred embodiment of the invention the warm-blooded animal is a human.

In a further preferred embodiment of the invention the therapeutically effective dose inhibits cell proliferation in the tumor.

A preferred subgroup of the COMPOUNDS OF THE INVENTION are the compounds of formula I as defined above wherein $R_4$ is 2-hydroxybenzyl or, preferably, 2-hydroxy-4-methoxybenzyl.

The invention relates in particular to COMPOUNDS OF THE INVENTION of formula I*,

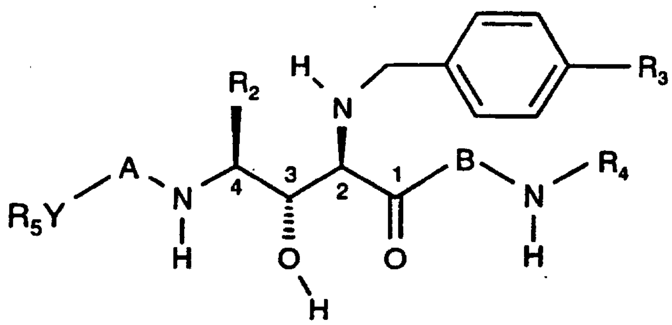

I*

wherein
A and B independently represent an unsubstituted or substituted amino acyl moiety;
$R_2$ represents aryl alkyl;
$R_3$ represents halogen, alkyl, alkoxy or hydroxy alkoxy;
$R_4$ represents 2-hydroxy-benzyl unsubstituted or substituted in 4 position by methoxy and
$R_5$ represents arylalkyl and Y represents -CO-; or
$R_5$ represents alkyl substituted by cycloalkyl, naphthyl, pyridyl or phenyl in which phenyl is substituted by alkyl or amino; and Y represents -O-CO-.

COMPOUNDS OF THE INVENTION of formula I* which are preferred are those, in which
A and B independently represent L-tert.-leucine, L-valine, L-glutaminic acid methyl ester or glycine;
$R_2$ represents arylalkyl;
$R_3$ represents alkoxy; and
$R_4$ represents 2-hydroxy-4-methoxybenzyl;
$R_5$ represents arylalkyl and Y represents -CO-; or
$R_5$ represents alkyl substituted by cycloalkyl, naphthyl, pyridyl or phenyl in which phenyl is substituted by alkyl or amino; and Y represents -O-CO-.

COMPOUNDS OF THE INVENTION of formula I* which are most preferred are those, in which
A and B independently represent L-tert.-leucine, L-valine, L-glutaminic acid methyl ester or glycine;
$R_2$ represents $C_7$-$C_{12}$arylalkyl;
$R_3$ represents $C_1$-$C_4$alkoxy;
$R_4$ represents 2-hydroxy-4-methoxybenzyl; and
$R_5$ represents $C_7$-$C_{12}$arylalkyl and Y represents -CO-; or
$R_5$ represents $C_1$-$C_4$alkyl substituted by $C_5$-$C_7$cycloalkyl, naphthyl, pyridyl or phenyl in which phenyl is substituted by $C_1$-$C_4$alkyl or amino; and Y represents -O-CO-.

Another preferred embodiment of the invention relates to the use of one of the following compounds or a pharmaceutically acceptable salt of one these compounds:

{1-[1-Benzyl-2-hydroxy-3-[1-(2-hydroxy-4-methoxy-benzylcarbamoyl)-2-methyl-propylcarbamoyl]-3-(4-methoxy-benzylamino)-propylcarbamoyl]-2-methyl-propyl}-carbamic acid benzyl ester;

N-{4(S)-[N-Benzyloxycarbonyl-tert-leucinoyl)amino]-3-(S)-hydroxy-2(R)-(4-methoxybenzylamino)-5-phenyl}pentanoyl-L-valine-N-[(2-hydroxy-4-methoxy)benzyl]amide; or

{1-[1-Benzyl-2-hydroxy-3-[1-(2-hydroxy-4-methoxy-benzylcarbamoyl)-2-methyl-propylcarbamoyl]-3-(4-methoxy-benzylamino)-propylcarbamoyl]-2,2-dimethyl-propyl}-carbamic acid tert-butyl ester.

Furthermore, the invention relates to a compound selected from the group of compounds consisting of 4-[4-(2-Benzyloxycarbonylamino-3-methyl-butyrylamino)-3-hydroxy-2-(4-methoxy-benzylamino)-5-phenyl-pentanoylamino]-4-(2-hydroxy-4-methoxy-benzylcarbamoyl)-butyric acid methyl ester;
{1-[1-Benzyl-2-hydroxy-3-[1-(2-hydroxy-4-methoxy-benzyl-carbamoyl)-2-methyl-propylcarbamoyl]-3-(4-methoxy-benzylamino)-propylcarbamoyl]-2,2-dimethyl-propyl}-carbamic acid naphthalen-1-ylmethyl ester;
{1-[1-Benzyl-2-hydroxy-3-[1-(2-hydroxy-4-methoxy-benzylcarbamoyl)-2-methyl-propylcarbamoyl]-3-(4-methoxy-benzylamino)-propylcarbamoyl]-2,2-dimethyl-propyl}-carbamic acid naphthalen-2-ylmethyl ester;
{1-[1-Benzyl-2-hydroxy-3-[1-(2-hydroxy-4-methoxy-benzylcarbamoyl)-2-methyl-propylcarbamoyl]-3-(4-methoxy-benzylamino)-propylcarbamoyl]-2,2-dimethyl-propyl}-carbamic acid pyridin-4-ylmethyl ester,
{[1-Benzyl-2-hydroxy-3-(1-(2-hydroxy-4-methoxy-benzylcarbamoyl)-2-methyl-propylcarbamoyl]-3-(4-methoxy-benzylamino)-propylcarbamoyl]-methyl}-carbamic acid benzyl ester; or
{1-[1-Benzyl-2-hydroxy-3-[1-(2-hydroxy-4-methoxy-benzylcarbamoyl)-2,2-dimethyl-propylcarbamoyl]-3-(4-methoxy-benzylamino)-propylcarbamoyl]-2,2-dimethyl-propyl}-carbamic acid benzyl ester;
4-[3,3-Dimethyl-2-(2-naphthalen-1-yl-acetylamino)-butyrylamino]-3-hydroxy-2-(4-methoxy-benzylamino)-5-phenyl-pentanoic acid [1-(2-hydroxy-4-methoxy-benzyl-carbamoyl)-2-methyl-propyl]-amide
{1-[1-Benzyl-2-hydroxy-3-[1-(2-hydroxy-4-methoxy-benzylcarbamoyl)-2-methyl-propylcarbamoyl]-3-(4-methoxy-benzylamino)-propylcarbamoyl]-2,2-dimethyl-propyl}-carbamic acid 3-methylbenzyl ester
4-{2-[3-(3-Amino-phenyl)-propionylamino]-3,3-dimethyl-butyrylamino}-3-hydroxy-2-(4-methoxy-benzylamino)-5-phenyl-pentanoic acid [1-(2-hydroxy-4-methoxy-benzylcarbamoyl)-2-methyl-propyl]-amide
{1-[1-Benzyl-2-hydroxy-3-[1-(2-hydroxy-4-methoxy-benzylcarbamoyl)-2-methyl-propylcarbamoyl]-3-(4-methoxy-benzylamino)-propylcarbamoyl]-2,2-dimethyl-propyl}-carbamic acid 3-amino-benzyl ester
{1-[1-Benzyl-2-hydroxy-3-[1-(2-hydroxy-4-methoxy-benzyl-carbamoyl)-2-methyl-propylcarbamoyl]-3-(4-methoxy-benzylamino)-propylcarbamoyl]-2,2-dimethyl-propyl}-carbamic acid 3,5-dimethyl-benzyl ester
or a pharmaceutically acceptable salt of one these compounds.

In a more preferred embodiment of the invention, the invention relates to 4-[4-(2-Benryloxycarbonylamino-3-methyl-butyrylamino)-3-hydroxy-2-(4-methoxy-benzylamino)-5-phenyl-pentanoylamino]-4-(2-hydroxy-4-methoxy-benzylcarbamoyl)-butyric acid methyl ester or a pharmaceutically acceptable salt of this compound.

Furthermore, the invention relates in particular to a compound selected from the group of compounds consisting of
4-[4-(2-Benzytoxycarbonylamino-3-methyl-butyrylamino)-3-hydroxy-2-(4-methoxy-benzylamino)-5-phenyl-pentanoylamino]-4-(2-hydroxy-4-methoxy-benzylcarbamoyl)-butyric acid methyl ester;
{1-[1-Benzyl-2-hydroxy-3-[1-(2-hydroxy-4-methoxy-benzyl-carbamoyl)-2-methyl-propylcarbamoyl]-3-(4-methoxy-benzylamino)-propylcarbamoyl]-2,2-dimethyl-propyl}-carbamic acid naphthalen-1-ylmethyl ester;
{1-[1-Benzyl-2-hydroxy-3-[1-(2-hydroxy-4-methoxy-benzylcarbamoyl)-2-methyl-propylcarbamoyl]-3-(4-methoxy-benzylamino)-propylcarbamoyl]-2,2-dimethyl-propyl}-carbamic acid naphthalen-2-ylmethyl ester;
{1-[1-Benzyl-2-hydroxy-3-[1-(2-hydroxy-4-methoxy-benzylcarbamoyl)-2-methyl-propylcarbamoyl]-3-(4-methoxy-benzylamino)-propylcarbamoyl]-2,2-dimethyl-propyl}-carbamic acid pyridin-4-ylmethyl ester;
{[1-Benzyl-2-hydroxy-3-[1-(2-hydroxy-4-methoxy-benzylcarbamoyl)-2-methyl-propylcarbamoyl]-3-(4-methoxy-benzylamino)-propylcarbamoyl]-methyl}-carbamic acid benzyl ester;
{1-[1-Benzyl-2-hydroxy-3-[1-(2-hydroxy-4-methoxy-benzylcarbamoyl)-2,2-dimethyl-propylcarbamoyl]-3-(4-methoxy-benzylamino)-propylcarbamoyl]-2,2-dimethyl-propyl}-carbamic acid benzyl ester; and
{1-[(1-Benzyl-2-hydroxy-3-[1-(2-hydroxy-4-methoxy-benzylcarbamoyl)-2-methyl-propylcarbamoyl]-3-(4-methoxy-benzylamino)-propylcarbamoyl]-2,2-dimethyl-propyl}-carbamic acid cyclohexylmethyl ester;
or a pharmaceutically acceptable salt of such a compound for use in a method for the therapeutic treatment of the human or animal body.

Furthermore, the invention relates to the use of a COMPOUND OF THE INVENTION for the treatment of a proliferative disease. The invention relates also to the use of COMPOUND OF THE INVENTION in the inhibition of the multicatalytic proteasome complex in warm-blooded animals, in particular humans.

The COMPOUNDS OF THE INVENTION may be admixed with conventional chemo-therapeutically acceptable diluents and carriers and administered e.g. parenterally or intravenously, preferably orally, in such forms as solutions, tablets or capsules. The concentrations of active substance will, of course, vary depending e.g. on the COMPOUND OF THE INVENTION employed, the treatment desired and the nature of the form.

Such compositions form part of the invention. The invention thus also relates to a pharmaceutical composition comprising a COMPOUND OF THE INVENTION of formula I* together with a pharmaceutical carrier, especially for the treatment of a proliferative disease.

Stable compositions for oral administration that offer high absorption efficiency and drug loading can be obtained by formulating an agent of the invention with a carrier medium comprising a hydrophilic phase, a lipophilic phase and a surfactant. Preferably the composition is in the form of a "microemulsion preconcentrate" or "emulsion preconcentrate", in particular of the type providing o/w (oil-in-water) microemulsions or emulsions. However, the composition may be in the form of a microemulsion or an emulsion which additionally contains an aqueous phase, preferably water. A "microemulsion preconcentrate" is a formulation which spontaneously forms a microemulsion in an aqueous medium, for example in water or in the gastric juices after oral application. A "microemulsion" is a non-opaque or substantially non-opaque colloidal dispersion that is formed spontaneously or substantially spontaneously when its components are brought into contact. A microemulsion is thermodynamically stable.

An "emulsion preconcentrate" is a formulation which spontaneously forms an emulsion in an aqueous medium, for example in water or in the gastric juices, after oral application. The emulsion formed is opaque and thermodynamically stable. The lipophilic phase may comprise 10 to 85 % by weight of the carrier medium; the surfactant may comprise 5 to 80 % by weight of the carrier medium; the hydrophilic phase may comprise 10 to 50 % by weight of the carrier medium.

The COMPOUND OF THE INVENTION is preferably present in an amount of about 1 to 15 % by weight of the composition, more preferably about 2 to 8 %.

The hydrophilic phase may be selected from e.g. Transcutol$^R$ ($C_2H_5$-[O-$(CH_2)_2$]$_2$-OH), Glycofurol$^R$ (also known as tetrahydrofurfuryl alcohol polyethylene glycol ether) and 1,2-propylene glycol, or mixtures thereof, and is preferably 1,2-propylene glycol. It may include further hydrophilic co-components, for example $C_1$-$C_5$alkanols.

Preferred lipophilic phase components are medium chain fatty acid triglycerides, mixed mono-, di-, tri-glycerides, and transesterified ethoxylated vegetable oils. Suitable medium chain fatty acid triglycerides are those known and commercially available under the trade names Miglyol$^R$, Captex$^R$, Captex$^R$, Neobee$^R$ and Mazol$^R$; Miglyol 812$^R$ being the most preferred. The mixed mono-, di-, tri-glycerides preferably comprise mixtures of $C_{12-20}$ fatty acid mono-, di- and tri-glycerides. The fatty acid component of the mixed mono-, di- and tri-glycerides may comprise both saturated and unsaturated fatty acid residues. The transesterified ethoxylated vegetable oils comprise transesterification products of, for example, almond oil, palm oil or, preferably, com oil, sunflower oil or safflower oil and most preferably com oil, with glycerol. In addition to their mono-, di- and tri-glyceride components, the transesterification products also generally comprise minor amounts of free glycerol. Preferably some of the glycerol is first removed to give a "substantially glycerol free batch" when soft gelatine capsules are to be made.

Trans-esterification products of com oil and glycerol provide particularly suitable mixed mono-, di-, and tri-glycerides. An example of a suitable mixed glyceride product is the trans-esterification product commercially available under the trade name Maisine$^R$ [available from Etablissements Gattefossé, 69804 Saint-Priest, Cedex (France)]. The fatty acid content for Maisine$^R$ is typically: about 11 % palmitic acid; about 2.5 % stearic acid; about 29 % oleic acid; about 56 % linoleic acid; and 1.5 % other acids.

If the agent of the invention is to be administered in the form of a microemulsion or emulsion, it is preferred that the mixed mono-, di-, and tri-glycerides are clear and remain clear for more than 20 days upon storage at temperatures of 20°C to 25°C. Also, a sample of the mixed mono-, di-, and tri-glycerides, which has been kept in a refrigerator at about between 2 and 8°C for 24 hours and then held at room temperature for 1 hour, should be dear. Also the mono-, di-, tri-glycerides preferably have a low saturated fatty acid content. Mixed mono-, di-, tri-glycerides meeting these requirements may be obtained from commercially available products by separation techniques as known in the art to remove the saturated fatty acid components and enhance the unsaturated fatty acid component content. Typically the total saturated fatty acid component will be less than 15 % by weight based on the total weight of the lipophilic phase. A suitable process is described in WO 93/09211.

The lipophilic phase may alternatively comprise suitable transesterified ethoxylated vegetable oils such as those obtained by reacting various natural vegetable oils with polyethylene glycols that have an average molecular weight of from 200 to 800, in the presence of an appropriate catalyst. The procedures are known and an example is described in US Patent 3 288 824. Transesterified ethoxylated com oil is particularly preferred. A preferred transesterified ethoxylated vegetable oil is Labrafil M 2125 CS$^R$.

Examples of suitable surfactants are:

i) reaction products of a natural or hydrogenated castor oil and ethylene oxide. The oils available under the trade name Cremophor$^R$ are especially suitable. Particularly suitable are Cremophor RH 40$^R$ and Cremophor RH60$^R$. Also suitable are polyethyleneglycol castor oils such as that available under the trade name Cremophor EL$^R$. Similar or identical products which may also be used are available under the trade names Nikkol$^R$, Mapeg$^R$, Incrocas$^R$ and Tagat$^R$.

ii) Polyoxyethylene-sorbitan-fatty acid esters, for example esters of the type known and commercially available under thetrade name Tween$^R$.

iii) Polyoxyethylene fatty acid esters, for example polyoxyethylene stearic acid esters of the type known and commercially available under the trade name Myrj$^R$.

iv) Polyoxyethylene-polyoxypropylene co-polymers and block co-polymers, e.g. of the type known and commercially available under the trade names Pluronic$^R$, Emkalyx$^R$ and Poloxamer$^R$, especially preferred are Pluronic F68$^R$ and Poloxamer 188$^R$.

v) Dioctylsulfosuccinate or di-[2-ethythexyl]-succinate.

vi) Phospholipids, in particular lecithins.

vii) Propylene glycol mono- and di-fatty acid esters.

The surfactant selected preferably has an HLB (hydrophilic/lipophilic balance) of at least 10.

Full physical characteristics of the products referred to herein by trade name can be obtained e.g. from H.P. Fiedler, "Lexikon der Hilfsstoffe für Pharmazie, Kosmetik und Angrenzende Gebiete", Editio Cantor, D-7960 Aulendorf, Germany, 3rd revised and expanded edition (1989). Preferably the relative proportion of hydrophilic phase component (s), the lipophilic phase and the surfactant lie within the "microemulsion" region on a standard three way plot. The compositions thus obtained are microemulsion preconcentrates of high stability.

Alternatively the components may be selected to provide an emulsion preconcentrate. The emulsion preconcentrate compositions also show good stability characteristics.

The pharmaceutical compositions may also include further additives or ingredients, for example antioxidants. They exhibit especially advantageous properties when administered orally, for example in terms of consistency and high level of bioavailability obtained in standard bioavailability trials. Pharmacokinetic parameters, for example absorption and blood levels, also become surprisingly more predictable and problems in administration with erratic absorption may be eliminate or reduced. Additionally the pharmaceutical composition is effective with tenside materials, for example bile salts, present in the gastro-intestinal tract.

The pharmaceutical compositions for oral use are preferably compounded in unit dosage form, for example by filling them into orally administrable capsule shells. The capsule shells may be soft or hard gelatine capsule shells. However, if desired the pharmaceutical compositions may be in a drink solution form and may include water or any other aqueous system, to provide emulsion or microemulsion systems suitable for drinking.

COMPOUNDS OF THE INVENTION are well tolerated at dosages required for the use and the methods in accordance with the present invention. Depending on the species, age, individual condition, mode of administration and the particular clinical picture, effective doses, for example daily doses of approximately 0.05 to about 10 g, preferably about 0.1 to about 5 g, for example about 0.15 g to 1.5 g, of a COMPOUND OF THE INVENTION are administered to a warm-blooded animal of approximately 70 kg body weight.

The present invention relates in particular to the use of COMPOUNDS OF THE INVENTION, and especially those of formula I*, in the manufacture of a pharmaceutical composition for the treatment of a proliferative disease, e. g. of a solid tumor and their use for the treatment of such a proliferative disease.

## Claims

**1.** Use of a 2,4-diamino-3-hydroxycarboxylic acid of formula I

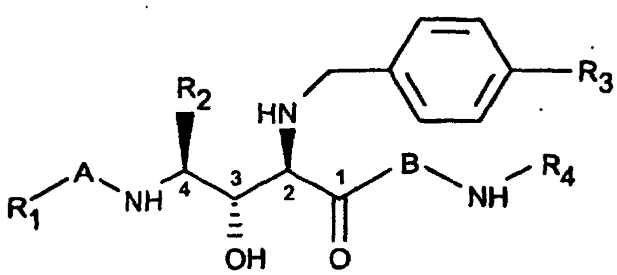

I

wherein

A and B independently represent a bond or an unsubstituted or substituted amino acyl moiety;

$R_1$ represents hydrogen; an amino protecting group; or a group of formula $R_5Y$- wherein

$R_5$ represents hydrogen or an unsubstituted or substituted alkyl, alkenyl, alkinyl, aryl, arylalkyl, heteroaryl, heteroarylalkyl, heterocyclyl or heterocyclyfalkyl group; and

Y represents -CO-; -NH-CO-; -NH-CS-; -$SO_2$-; -O-CO-; or -O-CS-;

$R_2$ represents the side chain of a natural amino acid; an alkyl, arylalkyl, heteroarylalkyl or cycloalkylalkyl group; or trimethylsilylmethyl, 2-thienylmethyl or styrylmethyl;
$R_3$ represents halogen, alkyl, alkoxy or hydroxyalkoxy; and
$R_4$ represents 2(R)-hydroxyindan-1 (S)-yl; (S)-2-hydroxy-1-phenylethyl; or 2-hydroxybenzyl unsubstituted or substituted in 4-position by methoxy;

in free form or in pharmaceutically acceptable salt or complex form in the manufacture of a pharmaceutical composition for the treatment of a proliferative disease responsive to an inhibition of the multicatalytic proteasome complex.

2. Use of a 2,4-diamino-3-hydroxycarboxylic acid of formula I according to claim 1, wherein A and B independently represent a bond or an amino acyl moiety, which is unsubstituted or substituted by alkyl or alkoxycarbonylalkyl;

$R_1$ represents hydrogen; an amino protecting group; or a group of formula $R_5$Y- wherein

$R_5$ represents hydrogen or an unsubstituted or substituted alkyl, alkenyl, alkinyl, aryl, arylalkyl, heteroaryl, heteroarylalkyl, heterocyclyl or heterocyclylalkyl group; and
Y represents -CO-; -NH-CO-; -NH-CS-; -$SO_2$-; -O-CO-; or -O-CS-;

$R_2$ represents the side chain of a natural amino acid; an alkyl, arylalkyl, heteroarylalkyl or cycloalkylalkyl group: or trimethylsilylmethyl, 2-thienylmethyl or styrylmethyl;
$R_3$ represents halogen, alkyl, alkoxy or hydroxyalkoxy; and
$R_4$ represents 2(R)-hydroxyindan-1(S)-yl; (S)-2-hydroxy-1-phenylethyl; or 2-hydroxybenzyl unsubstituted or substituted in 4 position by methoxy.

3. Use of a 2,4-diamino-3-hydroxycarboxylic acid of formula I according to claim 1, wherein

A and B independently represent a bond or an amino acyl moiety, which is unsubstituted or substituted by alkyl or alkoxycarbonylalkyl;
$R_1$ represents hydrogen; an amino protecting group; or a group of formula $R_5$Y- wherein

$R_5$ represents hydrogen or an unsubstituted or substituted alkyl, alkenyl, alkinyl, aryl, arylalkyl, heteroaryl, heteroarylalkyl, heterocyclyl or heterocyclylalkyl group; and
Y represents -CO-; -NH-CO-; -NH-CS-; -$SO_2$-; -O-CO-; or -O-CS-;

$R_2$ represents arylalkyl;
$R_3$ represents halogen, alkyl, alkoxy or hydroxyalkoxy; and
$R_4$ represents 2-hydroxybenzyl which is unsubstituted or substituted in 4 position by methoxy.

4. Use of a 2,4-diamino-3-hydroxycarboxylic acid of formula I according to claim 1, wherein

A and B independently represent an amino acyl moiety, which is unsubstituted or substituted by $C_1$-$C_4$alkyl or $C_1$-$C_4$alkoxycarbonyl $C_1$-$C_4$alkyl;
$R_1$ represents hydrogen or a group of formula $R_5$Y- wherein

$R_5$ represents hydrogen; $C_1$-$C_4$alkyl which is unsubstituted or substituted by phenoxy, hydroxy or amino; $C_2$-$C_4$alkenyl; $C_2$-$C_4$alkinyl; $C_6$-$C_{10}$aryl; $C_7$-$C_{12}$arylalkyl which is unsubstituted or substituted by hydroxy, $C_1$-$C_4$alkyl, amino or $C_1$-$C_4$alkyl amino; pyridyl $C_1$-$C_4$alkyl; and
Y represents -O-CO- or -CO-;

$R_2$ represents $C_7$-$C_{12}$arylalkyl;
$R_3$ represents halogen, $C_1$-$C_4$alkyl, $C_1$-$C_4$alkoxy or hydroxy $C_1$-$C_4$alkoxy; and
$R_4$ represents 2-hydroxybenzyl which is unsubstituted or substituted in 4 position by methoxy.

5. Use of a 2,4-diamino-3-hydroxycarboxylic acid of formula I according to claim 1, wherein A and B independently represent an amino acyl moiety, which is unsubstituted or substituted by $C_1$-$C_4$alkyl or $C_1$-$C_4$alkoxycarbonyl

$C_1$-$C_4$alkyl

$R_1$ represents hydrogen or a group of formula $R_5$Y- wherein

$R_5$ represents hydrogen; $C_1$-$C_4$alkyl which is unsubstituted or substituted by phenoxy, hydroxy or amino; $C_2$-$C_4$alkenyl; $C_2$-$C_4$alkinyl; $C_6$-$C_{10}$aryl; $C_7$-$C_{12}$arylalkyl which is unsubstituted or substituted by hydroxy, $C_1$-$C_4$alkyl, amino or $C_1$-$C_4$alkyl amino; pyridyl $C_1$-$C_4$alkyl; and
Y represents -O-CO- or -CO-;

$R_2$ represents $C_7$-$C_{12}$arylalkyl;
$R_3$ represents $C_1$-$C_4$alkoxy; and
$R_4$ represents 2-hydroxybenzyl substituted in 4 position by methoxy.

**6.** Use according to any one of the preceding claims wherein the proliferative disease is a tumor disease.

**7.** A compound of the formula I*,

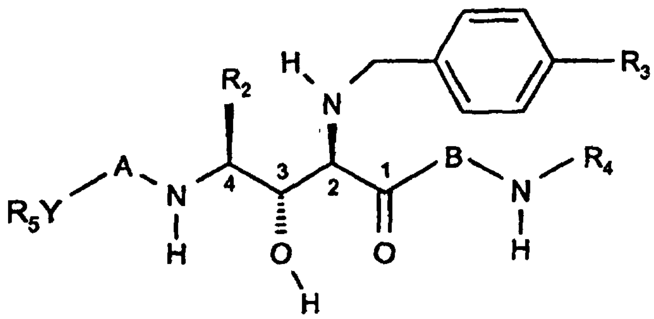

I*

wherein

A and B independently represent an unsubstituted or substituted amino acyl moiety;
$R_2$ represents arylalkyl;
$R_3$ represents halogen, alkyl, alkoxy or hydroxyalkoxy;
$R_4$ represents 2-hydroxy-benzyl unsubstituted or substituted in 4 position by methoxy; and
$R_5$ represents arylalkyl and
Y represents -CO-; or
$R_5$ represents alkyl substituted by cycloalkyl, naphthyl, pyridyl or phenyl in which phenyl is substituted by alkyl or amino; and
Y represents -O-CO-;

or a pharmaceutically acceptable salt thereof.

**8.** A compound of the formula I* according to claim 7, wherein

A and B independently represent L-tert.-leucine, L-valine, L-glutaminic acid methyl ester or glycine;
$R_2$ represents arylalkyl;
$R_3$ represents alkoxy;
$R_4$ represents 2-hydroxy-4-methoxybenzyl; and
$R_5$ represents arylalkyl and
Y represents -CO-; or
$R_5$ represents alkyl substituted by cycloalkyl, naphthyl, pyridyl or phenyl in which phenyl is substituted by alkyl or amino; and
Y represents -O-CO-;

or a pharmaceutically acceptable salt thereof.

**9.** A compound of the formula I* according to claim 7, wherein.

A and B independently represent L-tert.-leucine, L-valine, L-glutaminic acid methyl ester or glycine;
$R_2$ represents $C_7$-$C_{12}$arylalkyl;
$R_3$ represents $C_1$-$C_4$alkoxy;
$R_4$ represents 2-hydroxy-4-methoxybenzyl; and
$R_5$ represents $C_7$-$C_{12}$arylalkyl and
Y represents -CO-; or
$R_5$ represents $C_1$-$C_4$alkyl substituted by $C_5$-$C_7$cycloalkyl, naphthyl, pyridyl or phenyl in which phenyl is substituted by $C_1$-$C_4$alkyl or amino; and
Y represents -O-CO-;

or a pharmaceutically acceptable salt thereof.

**10.** A compound of formula I* according to claim 7 selected from the group of compounds consisting of
4-[4-(2-Benzyloxycarbonylamino-3-methyl-butyrylamino)-3-hydroxy-2-(4-methoxy-benzylamino)-5-phenyl-pentanoylamino]-4-(2-hydroxy-4-methoxy-benzylcarbamoyl)-butyric acid methyl ester,
{1-[1-Benzyl-2-hydroxy-3-[1-(2-hydroxy-4-methoxy-benzyl-carbamoyl)-2-methyl-propylcarbamoyl]-3-(4-methoxy-benzylamino)-propylcarbamoyl]-2,2-dimethyl-propyl}-carbamic acid naphthalen-1-ylmethyl ester;
{1-[1-Benzyl-2-hydroxy-3-[1-(2-hydroxy-4-methoxy-benzylcarbamoyl)-2-methyl-propylcarbamoyl]-3-(4-methoxy-benzylamino)-propylcarbamoyl]-2,2-dimethyl-propyl]}-carbamic acid naphthalen-2-ylmethyl ester;
{1-[1-Benzyl-2-hydroxy-3-[1-(2-hydroxy-4-methoxy-benzylcarbamoyl)-2-methyl-propylcarbamoyl]-3-(4-methoxy-benzylamino)-propylcarbamoyl]-2,2-dimethyl-propyl}-carbamic acid pyridin-4-ylmethyl ester;
{(1-Benzyl-2-hydroxy-3-[1-(2-hydroxy-4-methoxy-benzylcarbamoyl)-2-methyl-propylcarbamoyl]-3-(4-methoxy-benzylamino)-propylcarbamoyl]-methyl}-carbamic acid benzyl ester;
{1-[1-Benzyl-2-hydroxy-3-[1-(2-hydroxy-4-methoxy-benzylcarbamoyl)-2,2-dimethyl-propylcarbamoyl]-3-(4-methoxy-benzylamino)-propylcarbamoyl]-2,2-dimethyl-propyl}-carbamic acid benzyl ester,
and the pharmaceutically acceptable salts of these compounds.

**11.** A compound of formula I* according to claim 7, which is
4-[4-(2-Benzyloxycarbonylamino-3-methyl-butyrylamino)-3-hydroxy-2-(4-methoxy-benzylamino)-5-phenyl-pentanoylamino]-4-(2-hydroxy-4-methoxy-benzylcarbamoyl)-butyric acid methyl ester
or a pharmaceutically acceptable salt of this compound.

**12.** A compound of formula I* according to claim 7 selected from the group of compounds consisting of
4-[3,3-Dimethyl-2-(2-naphthalen-1-yl-acetylamino)-butyrylamino]-3-hydroxy-2-(4-methoxy-benzylamino)-5-phenyl-pentanoic acid [1-(2-hydroxy-4-methoxy-benzyl-carbamoyl)-2-methyl-propyl]-amide;
{1-[1-Benzyl-2-hydroxy-3-[1-(2-hydroxy-4-methoxy-benzylcarbamoyl)-2-methyl-propylcarbamoyl]-3-(4-methoxy-benzylamino)-propylcarbamoyl]-2,2-dimethyl-propyl}-carbamic acid 3-methylbenzyl ester;
4-{2-[3-(3-Amino-phenyl)-propionylamino]-3,3-dimethyl-butyrylamino}-3-hydroxy-2-(4-methoxy-benzylamino)-5-phenyl-pentanoic acid [1-(2-hydroxy-4-methoxy-benzylcarbamoyl)-2-methyl-propyl]-amide;
{1-[1-Benzyl-2-hydroxy-3-[1-(2-hydroxy-4-methoxy-benzylcarbamoyl)-2-methyl-propylcarbamoyl]-3-(4-methoxy-benzylamino)-propylcarbamoyl]-2,2-dimethyl-propyl}-carbamic acid 3-amino-benzyl ester;
{1-[1-Benzyl-2-hydroxy-3-[1-(2-hydroxy-4-methoxy-benzyl-carbamoyl)-2-methyl-propylcarbamoyl]-3-(4-methoxy-benzylamino)-propylcarbamoyl]-2,2-dimethyl-propyl}-carbamic acid 3,5-dimethyl-benzyl ester;
{1-[(1-Benzyl-2-hydroxy-3-[1-(2-hydroxy-4-methoxy-benzylcarbamoyl)-2-methyl-propylcarbamoyl]-3-(4-methoxy-benzylamino)-propylcarbamoyl]-2,2-dimethyl-propyl}-carbamic acid cyclohexylmethyl ester;
and the pharmaceutically acceptable salts of these compounds.

**13.** A compound of formula I* according to claim 7 or a pharmaceutically acceptable salt of such a compound for use as a medicament.

**14.** A pharmaceutical composition comprising a compound of formula I* according to claim 7 or a pharmaceutically acceptable salt of such a compound together with a pharmaceutical carrier.

## Patentansprüche

**1.** Verwendung einer 2,4-Diamino-3-hydroxycarbonsäure der Formel I

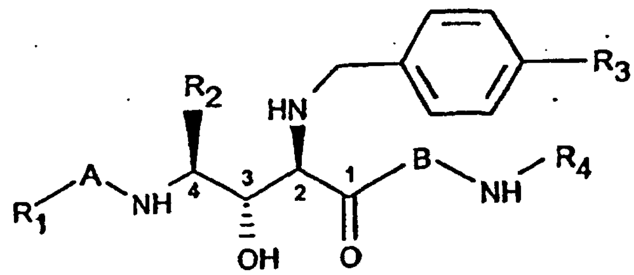

worin

A und B unabhängig für eine Bindung oder einen unsubstituierten oder substituierten Aminoacylrest stehen,
$R_1$ für Wasserstoff, eine Aminoschutzgruppe oder eine Gruppe der Formel $R_5Y$- steht, worin

$R_5$ für Wasserstoff oder eine unsubstituierte oder substituierte Alkyl-, Alkenyl-, Alkinyl-, Aryl-, Arylalkyl-, Heteroaryl-, Heteroarylalkyl-, Heterocyclyl- oder Heterocyclylalkylgruppe steht und
Y für -CO-, -NH-CO-, -NH-CS-, $-SO_2-$, -O-CO- oder -O-CS- steht,

$R_2$ für die Seitenkette einer natürlichen Aminosäure, eine Alkyl-, Arylalkyl-, Heteroarylalkyl- oder Cycloalkylalkylgruppe oder für Trimethylsilylmethyl, 2-Thienylmethyl oder Styrylmethyl steht,
$R_3$ für Halogen, Alkyl, Alkoxy oder Hydroxyalkoxy steht und
$R_4$ für 2(R)-Hydroxyindan-1-(S)-yl , (S)-2-Hydroxy-1-phenylethyl oder 2-Hydroxybenzyl steht, das unsubstituiert oder in Stellung 4 substituiert ist durch Methoxy,

in freier Form oder in Form eines pharmazeutisch annehmbaren Salzes oder Komplexes bei der Herstellung einer pharmazeutischen Zusammensetzung für die Behandlung einer proliferativen Erkrankung als Reaktion auf eine Hemmung des multikatalytischen Proteasomkomplexes.

**2.** Verwendung einer 2,4-diamino-3-hydroxycarbonsäure der Formel I nach Anspruch 1, worin
A und B unabhängig für eine Bindung oder einen Aminoacylrest stehen, der unsubstituiert oder substituiert ist durch Alkyl oder Alkoxycarbonylalkyl,

$R_1$ für Wasserstoff, eine Aminoschutzgruppe oder eine Gruppe der Formel $R_5Y$- steht, worin

$R_5$ für Wasserstoff oder eine unsubstituierte oder substituierte Alkyl-, Alkenyl-, Alkinyl-, Aryl-, Arylalkyl-, Heteroaryl-, Heteroarylalkyl-, Heterocyclyl- oder Heterocyclylalkylgruppe steht und
Y für -CO-, -NH-CO-, -NH-CS- , $-SO_2-$, -O-CO- oder -O-CS- steht,

$R_2$ für die Seitenkette einer natürlichen Aminosäure, eine Alkyl-, Arylalkyl-, Heteroarylalkyl- oder Cycloalkylalkylgruppe oder für Trimethylsilylmethyl, 2-Thienylmethyl oder Styrylmethyl steht,
$R_3$ für Halogen, Alkyl, Alkoxy oder Hydroxyalkoxy steht und
$R_4$ für 2(R)-Hydroxyindan-1-(S)-yl , (S)-2-Hydroxy-1-phenylethyl oder 2-Hydroxybenzyl steht, das unsubstituiert oder in Stellung 4 substituiert ist durch Methoxy.

**3.** Verwendung einer 2,4-Diamino-3-hydroxycarbonsäure der Formel I nach Anspruch 1, worin
A und B unabhängig für eine Bindung oder einen Aminoacylrest stehen, der unsubstituiert oder substituiert ist durch Alkyl oder Alkoxycarbonylalkyl,

$R_1$ für Wasserstoff, eine Aminoschutzgruppe oder eine Gruppe der Formel $R_5Y$- steht, worin

$R_5$ für Wasserstoff oder eine unsubstituierte oder substituierte Alkyl-, Alkenyl-, Alkinyl-, Aryl-, Arylalkyl-, Heteroaryl-, Heteroarylalkyl-, Heterocyclyl- oder Heterocyclylalkylgruppe steht und
Y für -CO-, -NH-CO-, -NH-CS-, $-SO_2-$, -O-CO- oder -O-CS- steht,

$R_2$ für Arylalkyl steht,

$R_3$ für Halogen, Alkyl, Alkoxy oder Hydroxyalkoxy steht und
$R_4$ für 2-Hydroxybenzyl steht, das unsubstituiert oder in Stellung 4 substituiert ist durch Methoxy.

**4.** Verwendung einer 2,4-Diamino-3-hydroxycarbonsäure der Formel 1 nach Anspruch 1, worin A und B unabhängig für einen Aminoacylrest stehen, der unsubstituiert oder substituiert ist durch $C_1$-$C_4$-Alkyl oder $C_1$-$C_4$-Alkoxycarbonyl-$C_1$-$C_4$-alkyl,

$R_1$ für Wasserstoff oder eine Gruppe der Formel $R_5Y$- steht, worin

$R_5$ für Wasserstoff, $C_1$-$C_4$-Alkyl, das unsubstituiert oder substituiert ist durch Phenoxy, Hydroxy oder Amino, für $C_2$-$C_4$-Alkenyl, $C_2$-$C_4$-Alkinyl, $C_6$-$C_{10}$-Aryl, $C_7$-$C_{12}$-Arylalkyl, das unsubstituiert oder substituiert ist durch Hydroxy, $C_1$-$C_4$-Alkyl, Amino oder $C_1$-$C_4$-Alkylamino, oder für Pyridyl- $C_1$-$C_4$-alkyl steht und
Y für -O-CO- oder -CO- steht,

$R_2$ für $C_7$-$C_{12}$-Arylalkyl steht,
$R_3$ für Halogen, $C_1$-$C_4$-Alkyl, $C_1$-$C_4$-Alkoxy oder Hydroxy- $C_1$-$C_4$-alkoxy steht und
$R_4$ für 2-Hydroxybenzyl steht, das unsubstituiert oder in Stellung 4 substituiert ist durch Methoxy.

**5.** Verwendung einer 2,4-Diamino-3-hydroxycarbonsäure der Formel I nach Anspruch 1 worin A und B unabhängig für einen Aminoacylrest stehen, der unsubstituiert oder substituiert ist durch $C_1$-$C_4$-Alkyl oder $C_1$-$C_4$-Alkoxycarbonyl-$C_1$-$C_4$-alkyl,

$R_1$ für Wasserstoff oder eine Gruppe der Formel $R_5Y$- steht, worin

$R_5$ für Wasserstoff, $C_1$-$C_4$-Alkyl, das unsubstituiert oder substituiert ist durch Phenoxy, Hydroxy oder Amino, für $C_2$-$C_4$-Alkenyl, $C_2$-$C_4$-Alkinyl, $C_6$-$C_{10}$-Aryl, $C_7$-$C_{12}$-Arylalkyl, das unsubstituiert oder substituiert ist durch Hydroxy, $C_1$-$C_4$-Alkyl, Amino oder $C_1$-$C_4$-Alkylamino, oder für Pyridyl- $C_1$-$C_4$-alkyl steht und
Y für -O-CO- oder -CO- steht,

$R_2$ für $C_7$-$C_{12}$-Arylalkyl steht,
$R_3$ für $C_1$-$C_4$-Alkoxy steht und
$R_4$ für 2-Hydroxybenzyl steht, das in Stellung 4 substituiert ist durch Methoxy.

**6.** Verwendung nach einem der vorhergehenden Ansprüche, worin die proliferative Erkrankung eine Tumorerkrankung ist.

**7.** Verbindung der Formel I*

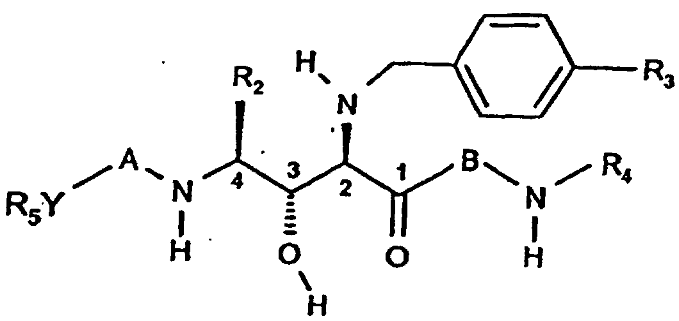

worin

A und B unabhängig für eine Bindung oder einen unsubstituierten oder substituierten Aminoacylrest stehen,
$R_2$ für Arylalkyl steht,
$R_3$ für Halogen, Alkyl, Alkoxy oder Hydroxyalkoxy steht,
$R_4$ für 2-Hydroxybenzyl steht, das unsubstituiert oder in Stellung 4 substituiert ist durch Methoxy und
$R_5$ für Arylalkyl steht und
Y für -CO- steht, oder

$R_5$ für Alkyl steht, das substituiert ist durch Cycloalkyl, Naphthyl, Pyridyl oder Phenyl, worin Phenyl substituiert ist durch Alkyl oder Amino, und
Y für -O-CO- steht,

oder ein pharmazeutisch annehmbares Salz hiervon.

**8.** Verbindung der Formel I* nach Anspruch 7, worin

A und B unabhängig für L-tert-Leucin, L-Valin, L-Glutaminsäuremethylester oder Glycin stehen,
$R_2$ für Arylalkyl steht,
$R_3$ für Alkoxy steht,
$R_4$ für 2-Hydroxy-4-methoxybenzyl steht und
$R_5$ für Arylalkyl steht und
Y für -CO- steht, oder
$R_5$ für Alkyl steht, das substituiert ist durch Cycloalkyl, Naphtyl, Pyridyl oder Phenyl, worin Phenyl substituiert ist durch Alkyl oder Amino, und
Y für -O-CO- steht,

oder ein pharmazeutisch annehmbares Salz hiervon.

**9.** Verbindung der Formel I* nach Anspruch 7, worin

A und B unabhängig für L-tert-Leucin, L-Valin, L-Glutaminsäuremethylester oder Glycin stehen,
$R_2$ für $C_7$-$C_{12}$-Arylalkyl steht,
$R_3$ für $C_1$-$C_4$-Alkoxy steht,
$R_4$ für 2-Hydroxy-4-methoxybenryl steht und
$R_5$ für $C_7$-$C_{12}$-Arylalkyl und
Y für -CO- steht, oder
$R_5$ für $C_1$-$C_4$-Alkyl steht, das substituiert ist durch $C_5$-$C_7$-Cycloalkyl, Naphthyl, Pyridyl oder Phenyl, worin Phenyl substituiert ist durch $C_1$-$C_4$-Alkyl oder Amino, und
Y für -O-CO- steht,

oder ein pharmazeutisch annehmbares Salz hiervon.

**10.** Verbindung der Formel I* nach Anspruch 7, ausgewählt aus der Gruppe von Verbindungen, die besteht aus
4-[4-(2-Benzyloxycarbonylamino-3-methyl-butyrylamino)-3-hydroxy-2-(4-methoxy-benzylamino)-5-phenyl-pentanoylamino]-4-(2-hydroxy-4-methoxy-benzylcarbamoyl)-buttersäuremethylester,
{1-[1-Benzyl-2-hydroxy-3-[1-(2-hydroxy-4-methoxy-benzylcarbamoyl)-2-methyl-propylcarbamoyl]-3-(4-methoxy-benzylamino)-propylcarbamoyl]-2,2-dimethyl-propyl}-carbaminsäurenaphthalin-1-ylmethylester,
{1-[1-Benzyl-2-hydroxy-3-[1-(2-hydroxy-4-methoxy-benzylcarbamoyl)-2-methyl-propylcarbamoyl]-3-(4-methoxy-benzylamino)-propylcarbamoyl]-2,2-dimethyl-propyl}-carbaminsäurenaphthalin-2-ylmethylester,
{1-[1-Benzyl-2-hydroxy-3-[1-(2-hydroxy-4-methoxy-benzylcarbamoyl)-2-methyl-propylcarbamoyl]-3-(4-methoxy-benzylamino)-propylcarbamoyl]-2,2-dimethyl-propyl}-carbaminsäurepyridin-4-ylmethylester,
{[1-Benzyl-2-hydroxy-3-[1-(2-hydroxy-4-methoxy-benzylcarbamoyl)-2-methyl-propylcarbamoyl]-3-(4-methoxy-benzylamino)-propylcarbamoyl]-methyl}-carbaminsäurebenzylester,
{1-[1-Benzyl-2-hydroxy-3-[1-(2-hydroxy-4-methoxy-benzylcarbamoyl)-2,2-dimethyl-propylcarbamoyl]-3-(4-methoxy-benzylamino)-propylcarbamoyl]-2,2-dimethyl-propyl}-carbaminsäurebenzylester,
und die pharmazeutisch annehmbaren Salze dieser Verbindungen.

**11.** Verbindung der Formel I* nach Anspruch 7, nämlich
4-[4-(2-Benzyloxycarbonylamino-3-methyl-butyrylamino)-3-hydroxy-2-(4-methoxy-benrylamino)-5-phenyl-pentanoylamino]-4-(2-hydroxy-4-methoxy-benzylcarbamoyl)-buttersäuremethylester
oder ein pharmazeutisch annehmbares Salz dieser Verbindung.

**12.** Verbindung der Formel I* nach Anspruch 7, die ausgewählt ist aus der Gruppe von Verbindungen, die besteht aus
4-[3,3-Dimethyl-2-(2-naphthalin-1-ylacetylamino)-butyrylamino]-3-hydroxy-2-(4-methoxy-benzylamino)-5-phenyl-pentansäure [1-(2-hydroxy-4-methoxy-benzylcarbamoyl)-2-methyl-propyl]-amid,
{1-[1-Benzyl-2-hydroxy-3-[1-(2-hydroxy-4-methoxy-benzylcarbamoyl)-2-methyl-propylcarbamoyl]-3-(4-methoxy-benzylamino)-propylcarbamoyl]-2,2-dimethyl-propyl}-carbaminsäure-3-methylbenzylester,

4-{2-[3-(3-Amino-phenyl)-propionylamino]-3,3-dimethyl-butyrylamino}-3-hydroxy-2-(4-methoxy-benzylamino)-5-phenyl-pentansäure-[1-(2-hydroxy-4-methoxy-benzylcarbamoyl)-2-methyl-propyl]-amid,

{1-[1-Benzyl-2-hydroxy-3-[1-(2-hydroxy-4-methoxy-benzylcarbamoyl)-2-methyl-propylcarbamoyl]-3-(4-methoxy-benzylamino)-propylcarbamoyl]-2,2-diemethyl-propyl}-carbaminsäure-3-aminobenzylester,

{1-[1-Benzyl-2-hydroxy-3-[1-(2-hydroxy-4-methoxy-benzylcarbamoyl)-2-methyl-propylcarbamoyl]-3-(4-methoxy-benzylamino)-propylcarbamoyl]-2,2-dimethyl-propyl}-carbaminsäure-3,5-dimethylbenzylester,

{1-[(1-Benzyl-2-hydroxy-3-[1-(2-hydroxy-4-methoxy-benzylcarbamoyl)-2-methyl-propylcarbamoyl]-3-(4-methoxy-benzylamino)-propylcarbamoyl]-2,2-dimethyl-propyl}-carbaminsäurecyclohexylmethylester,

und die pharmazeutisch annehmbaren Salze dieser Verbindungen.

**13.** Verbindung der Formel I* nach Anspruch 7 oder ein pharmazeutisch annehmbares Salz einer solchen Verbindung zur Verwendung als Arzneimittel.

**14.** Pharmazeutische Zusammensetzung, enthaltend eine Verbindung der Formel I* nach Anspruch 7 oder ein pharmazeutisch annehmbares Salz einer solchen Verbindung zusammen mit einem pharmazeutischen Träger.

## Revendications

**1.** Utilisation d'un acide 2,4-diamino-3-hydroxycarboxylique de formule I

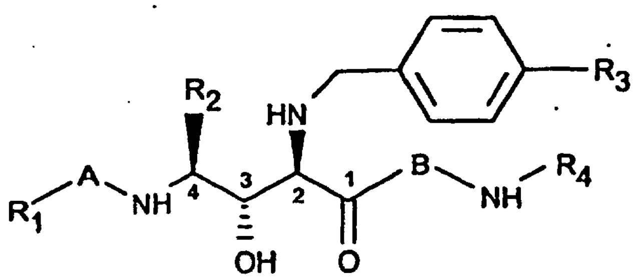

I

où,

A et B, indépendamment l'un de l'autre, représentent une liaison ou un groupe aminoacyle substitué ou non substitué ;

$R_1$ représente l'hydrogène ; un groupe amino protecteur ou un groupe de formule $R_5Y$- où,

$R_5$ représente l'hydrogène ou un groupe alkyle, alkényle, alkinyle, aryle, arylalkyle, hétéroaryle, hétéroarylalkyle, hétérocyclyle ou hétérocyclylalkyle substitué ou non substitué ; et

Y représente -CO- ; -NH-CO- ; -NH-CS- ; $-SO_2-$ ; -O-CO- ou -O-CS-

$R_2$ représente la chaîne latérale d'un amino acide naturel ; un groupe alkyle, arylalkyle, hétéroarylalkyle ou cycloalkylalkyle ; ou triméthylsilylméthyle, 2-thiénylméthyle ou styrylméthyle ;

$R_3$ représente halogène, alkyle, alcoxy ou hydroxyalcoxy ; et

$R_4$ représente 2(R)-hydroxyindan-1(S)-yl ; (S)-2-hydroxy-1-phényléthyle ou 2-hydroxy-benzyle non substitué ou substitué en position 4 par méthoxy ;

sous forme libre ou sous forme de complexe ou de sel pharmaceutiquement acceptable pour la fabrication d'une composition pharmaceutique destinée au traitement d'une maladie proliférative répondant à l'inhibition du complexe protéasome multicatalytique.

**2.** Utilisation d'un acide 2,4-diamino-3-hydroxycarboxylique de formule I selon la revendication 1 où

A et B, indépendamment l'un de l'autre, représentent une liaison ou un groupe aminoacyle non substitué ou substitué par alkyle ou alcoxycarbonylalkyle ;

$R_1$ représente l'hydrogène ; un groupe amino protecteur ou un groupe de de formule $R_5Y$- où,

$R_5$ représente l'hydrogène ou un groupe alkyle, alkényle, alkinyle, aryle, arylalkyle, hétéroaryle, hétéroarylalkyle, hétérocyclyle ou hétérocyclylalkyle substitué ou non substitué ; et

Y représente -CO- ; -NH-CO- ; -NH-CS- ; $-SO_2-$ ; -O-CO- ou -O-CS-;

$R_2$ représente la chaîne latérale d'un amino acide naturel ; un groupe alkyle, arylalkyle, hétéroarylalkyle ou cycloalkylalkyle ; triméthylsilylméthyle, 2-thiénylméthyle ou styrylméthyle ;

$R_3$ représente halogène, alkyle, alcoxy ou hydroxyalcoxy ; et
$R_4$ représente 2(R)-hydroxyindan-1(S)-yl ; (S)-2-hydroxy-1-phényléthyle ou 2-hydroxy-benzyle non substitué ou substitué en position 4 par méthoxy ;

**3.** Utilisation d'un acide 2,4-diamino-3-hydroxycarboxylique de formule I selon la revendication 1 où
A et B, indépendamment l'un de l'autre, représentent une liaison ou un groupe aminoacyle non substitué ou substitué par alkyle ou alcoxycarbonylalkyle ;
$R_1$ représente l'hydrogène ; un groupe amino protecteur ou un groupe de formule $R_5$Y- où,
$R_5$ représente l'hydrogène ou un groupe alkyle, alkényle, alkinyle, aryle, arylalkyle, hétéroaryle, hétéroarylalkyle, hétérocyclyle ou hétérocyclylalkyle substitué ou non substitué ; et
Y représente -CO- ; -NH-CO- ; -NH-CS- ; -$SO_2$- ; -O-CO- ou -O-CS-;
$R_2$ représente arylalkyle ;
$R_3$ représente halogène, alkyle, alcoxy ou hydroxyalcoxy ; et
$R_4$ représente 2-hydroxybenzyle non substitué ou substitué en position 4 par méthoxy.

**4.** Utilisation d'un acide 2,4-diamino-3-hydroxycarboxylique de formule I selon la revendication 1 où
A et B, indépendamment l'un de l'autre, représentent une liaison ou un groupe aminoacyle non substitué ou substitué par $C_1$-$C_4$alkyle ou $C_1$-$C_4$alcoxycarbonyle $C_1$-$C_4$alkyle ;
$R_1$ représente l'hydrogène ou un groupe de formule $R_5$Y- où,
$R_5$ représente l'hydrogène; $C_1$-$C_4$alkyle non substitué ou substitué par phénoxy, hydroxy ou amino ; $C_2$-$C_4$alkényle ; $C_2$-$C_4$alkinyle ; $C_6$-$C_{10}$aryle ; $C_7$-$C_{12}$arylalkyle non substitué ou substitué par hydroxy, $C_1$-$C_4$alkyle, amino ou $C_1$-$C_4$alkylamino ; pyridyl $C_1$-$C_4$alkyle ; et
Y représente -O-CO- ou -CO- ;
$R_2$ représente $C_7$-$C_{12}$arylalkyle ;
$R_3$ représente halogène, $C_1$-$C_4$alkyle, $C_1$-$C_4$alcoxy ou hydroxy $C_1$-$C_4$alcoxy ; et
$R_4$ représente 2-hydroxybenzyle non substitué ou substitué en position 4 par méthoxy.

**5.** Utilisation d'un acide 2,4-diamino-3-hydroxycarboxylique de formule I selon la revendication 1 où
A et B, indépendamment l'un de l'autre, représentent un groupe aminoacyle non substitué ou substitué par $C_1$-$C_4$alkyle ou $C_1$-$C_4$alcoxycarbonyle$C_1$-$C_4$alkyle ;
$R_1$ représente l'hydrogène ou un groupe de formule $R_5$Y- où,
$R_5$ représente l'hydrogène; $C_1$-$C_4$alkyle non substitué ou substitué par phénoxy, hydroxy ou amino ; $C_2$-$C_4$alkényle ; $C_2$-$C_4$alkinyle ; $C_6$-$C_{10}$aryle ; $C_7$-$C_{12}$arylalkyle non substitué ou substitué par de l'hydroxy, $C_1$-$C_4$alkyle, amino ou $C_1$-$C_4$alkylamino ; pyridyl $C_1$-$C_4$alkyle ;
Y représente -O-CO- ou -CO- ;
$R_2$ représente $C_7$-$C_{12}$arylalkyle ;
$R_3$ représente $C_1$-$C_4$alcoxy ; et
$R_4$ représente 2-hydroxybenzyle substitué en position 4 par méthoxy.

**6.** Utilisation selon l'une quelconque des revendications précédentes **caractérisée en ce que** la maladie proliférative est une tumeur.

**7.** Composé de formule 1 *

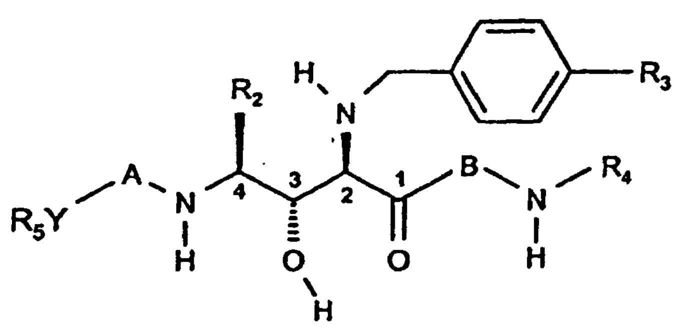

I*

où,
A et B, indépendamment l'un de l'autre, représentent un groupe aminoacyle

substitué ou non substitué ;
$R_2$ représente arylalkyle ;
$R_3$ représente halogène, alkyle, alcoxy ou hydroxyalcoxy ;
$R_4$ représente 2-hydroxy-benzyle non substitué ou substitué en position 4 par méthoxy ; et
$R_5$ représente arylalkyle ; et
Y représente -CO- ; ou
$R_5$ représente alkyle substitué par cycloalkyle, naphtyle, pyridyle ou phényle lequel phényle est substitué par alkyle ou amino ; et
Y représente -O-CO- ;
ou un de ses sels pharmaceutiquement acceptables.

**8.** Composé de formule I* selon la revendication 7 où,
A et B, indépendamment l'un de l'autre, représentent la L-tert.-leucine, la L-valine, l'ester méthylique de l'acide L-glutamique ou la glycine ;
$R_2$ représente arylalkyle ;
$R_3$ représente alcoxy ;
$R_4$ représente 2-hydroxy-4-méthoxybenzyle ; et
$R_5$ représente arylalkyle et
Y représente -CO- ; ou
$R_5$ représente alkyle substitué par cycloalkyle, naphtyle, pyridyle ou phényle lequel phényle est substitué par alkyle ou amino ; et
Y représente -O-CO- ;
ou un de ses sels pharmaceutiquement acceptables.

**9.** Composé de formule I* selon la revendication 7 où,
A et B, indépendamment l'un de l'autre, représentent la L-tert.-leucine, la L-valine, l'ester méthylique de l'acide L-glutamique ou la glycine ;
$R_2$ représente $C_7$-$C_{12}$arylalkyle ;
$R_3$ représente $C_1$-$C_4$alcoxy ;
$R_4$ représente 2-hydroxy-4-méthoxybenzyle ; et
$R_5$ représente $C_7$-$C_{12}$arylalkyle et
Y représente -CO- ; ou
$R_5$ représente $C_1$-$C_4$alkyle substitué par $C_5$-$C_7$cycloalkyle, naphtyle, pyridyle ou phényle lequel phényle est substitué par $C_1$-$C_4$alkyle ou amino ; et
Y représente -O-CO-
ou un de ses sels pharmaceutiquement acceptables.

**10.** Composé de formule I* selon la revendication 7 choisi parmi le groupe de composés consistant en
Ester méthylique de l'acide 4-[4-(2-benzyloxycarbonylamino-3-méthyl-butyrylamino)-3-hydroxy-2-(4-méthoxy-benzylamino)-5-phényl-pentanoylamino]-4-(2-hydroxy-4-méthoxy-benzylcarbamoyl)-butyrique ;
Ester naphthalèn-1-ylméthylique de l'acide {1-[1-benzyl-2-hydroxy-3-[1-(2-hydroxy-4-méthoxy-benzylcarbamoyl)-2-méthyl-propylcarbamoyl]-3-(4-méthoxy-benzylamino)-propylcarbamoyl]-2,2-diméthyl-propyl}-carbamique ;
Ester naphthalèn-2-ylméthylique de l'acide {1-[1-benzyl-2-hydroxy-3-[1-(2-hydroxy-4-méthoxy-benzylcarbamoyl)-2-méthyl-propylcarbamoyl]-3-(4-méthoxy-benzylamino)-propylcarbamoyl]-2,2-diméthyl-propyl}-carbamique ;
Ester pyridin-4-ylméthylique de l'acide {1-[1-benzyl-2-hydroxy-3-[1-(2-hydroxy-4-méthoxy-benzylcarbamoyl)-2-méthyl-propylcarbamoyl]-3-(4-méthoxy-benzylamino)-propylcarbamoyl]-2,2-diméthyl-propyl}-carbamique ;
Ester benzylique de l'acide {[1-benzyl-2-hydroxy-3-[1-(2-hydroxy-4-méthoxy-benzylcarbamoyl)-2-méthyl-propylcarbamoyl]-3-(4-méthoxy-benzylamino)-propylcarbamoyl]-méthyl}-carbamique ;
Ester benzylique de l'acide {1-[1-benzyl-2-hydroxy-3-[1-(2-hydroxy-4-méthoxy-benzylcarbamoyl)-2,2-diméthyl-propylcarbamoyl]-3-(4-méthoxy-benzylamino)-propyl-carbamoyl]-2,2-diméthyl-propyl}-carbamique ;
et les sels pharmaceutiquement acceptables de ces composés.

**11.** Composé de formule I* selon la revendication 7 qui est l'ester méthylique de l'acide 4-[4-(2-benzyloxycarbonylamino-3-méthyl-butyrylamino)-3-hydroxy-2-(4-méthoxy-benzylamino)-5-phényl-pentanoylamino]-4-(2-hydroxy-4-méthoxy-benzylcarbamoyl)-butyrique

ou un de ses sels pharmaceutiquement acceptables.

**12.** Composé de formule I* selon la revendication 7 choisi parmi le groupe de composés consistant en

[1-(2-Hydroxy-4-méthoxy-benzylcarbamoyl)-2-méthyl-propyl]-amide de l'acide 4-[3,3-diméthyl-2-(2-naphtalèn-1-yl-acétylamino)-butyrylamino]-3-hydroxy-2-(4-méthoxy-benzylamino)-5-phenyl-pentanoïque ;

Ester 3-méthylbenzylique de l'acide {1-[1-benzyl-2-hydroxy-3-[1-(2-hydroxy-4-méthoxy-benzylcarbamoyl)-2-méthyl-propylcarbamoyl]-3-(4-méthoxy-benzylamino)-propylcarbamoyl]-2,2-diméthyl-propyl}-carbamique ;

[1-(2-Hydroxy-4-méthoxy-benzylcarbamoyl)-2-méthyl-propyl]-amide de l'acide 4-{2-[3-(3-amino-phényl)-propionylamino]-3,3diméthyl-butyrylamino}-3-hydroxy-2-(4-méthoxy-benzylamino)-5-phényl-pentanoïque ;

Ester 3-amino-benzylique de l'acide {1-[1-benzyl-2-hydroxy-3-[1-(2-hydroxy-4-méthoxy-benzylcarbamoyl)-2-méthyl-propylcarbamoyl]-3-(4-méthoxy-benzylamino)-propylcarbamoyl]-2,2-diméthyl-propyl}-carbamique ;

Ester 3,5-diméthyl-benzylique de l'acide {1-[1-benzyl-2-hydroxy-3-[1-(2-hydroxy-4-méthoxy-benzylcarbamoyl)-2-méthyl-propylcarbamoyl]-3-(4-méthoxy-benzylamino)-propylcarbamoyl]-2,2-diméthyl-propyl}-carbamique ;

Ester cyclohexylméthylique de l'acide {1-[1-benzyl-2-hydroxy-3-[1-(2-hydroxy-4-méthoxy-benzylcarbamoyl)-2-méthyl-propylcarbamoyl]-3-(4-méthoxy-benzylamino)-propylcarbamoyl]-2,2-diméthyl-propyl}-carbamique ;

et les sels pharmaceutiquement acceptables de ces composés.

**13.** Composé de formule I* selon la revendication 7 ou un sel pharmaceutiquement acceptable de ce composé pour utilisation comme médicament.

**14.** Composition pharmaceutique comprenant un composé de formule I* selon la revendication 7 ou un sel pharmaceutiquement acceptable de ce composé associé à un véhicule pharmaceutique.